# EUROPÄISCHE PATENTSCHRIFT

(11) **EP 0 387 775 B1**
(45) Veröffentlichungstag und Bekanntmachung des Hinweises auf die Patenterteilung: **31.07.1996**
(21) Anmeldenummer: 90104701.9
(22) Anmeldetag: 13.03.1990
(51) Int. Cl.: C12N 15/11, C12N 9/00

(54) **Genetische Einheiten zur Inhibierung der Funktion von RNA**
Genetic construct for inhibiting RNA function
Unité génétique pour inhiber la fonction d'ARN

(30) Priorität: 16.03.1989 AT 609/89
(43) Veröffentlichungstag der Anmeldung: 19.09.1990
(73) Patentinhaber: BOEHRINGER INGELHEIM INTERNATIONAL GmbH, D-55216 Ingelheim am Rhein (DE)
(72) Erfinder: Beug, Hartmut, Dr., A-1140 Wien (AT); Birnstiel, Max L., Prof.-Dr., A-1100 Wien (AT); Cotten, Matthew, Dr., A-1130 Wien (AT); Wagner, Ernst, Dr., A-2103 Langenzerdorf (AT); Kandolf, Harald, Dipl.-Ing., A-1060 Wien (AT)

(56) Entgegenhaltungen:
- WO-A-88/04300
- THE EMBO JOURNAL, Band 6, Nr. 10, 1987, IRL Press Ltd., Oxford (GB); P.A. JENNINGS et al., Seiten 3043-3047
- NATURE, Band 334, 18 August 1988; J. HASELOFF et al., Seiten 585-591
- BIOTECHNIQUES, Band 6, 1988; J.N.M. MOL et al., Seiten 958-976
- SCIENTIFIC AMERICAN, Band 255, Nr. 5, November 1986, New York, NY (US); T.R. CECH, Seiten 76-84
- THE EMBO JOURNAL, Band 8, Nr. 12, 1989; M. COTTEN et al., Seiten 3861-3866

## Beschreibung

Die vorliegende Erfindung bezieht sich auf die spezifische Inhibierung von RNA durch Wechselwirkung mit RNA.

Die spezifische Inhibierung von Genen durch Oligonukleotide, z.B. im Hinblick auf eine therapeutisch zu bewirkende Blockierung von deregulierten Onkogenen oder viralen Genen, beruht auf der Fähigkeit solcher komplementärer RNA- oder DNA-Sequenzen, sog. "Antisense-Oligonukleotide", mit mRNAs, Prozessierungssignalen oder pre-mRNAs zu hybridisieren und auf diese Weise die Informationsübertragung von Genen auf Proteine zu unterbrechen.

Die Anwendung von Antisense-DNA bewirkt einen Abbau von komplementärer Ziel-RNA durch RNAse H-Spaltung des entstandenen Hybrids, das Ergebnis ist die irreversible Zerstörung der jeweiligen komplementären RNA.

Im Fall der Anwendung von Antisense-RNA erfolgt ein sog. "hybrider Arrest" von Translation oder Prozessierung, wobei die RNA/RNA Hybride das strukturelle Hindernis darstellen. Es wird angenommen, daß solche Hybride in der Zelle akkumulieren; ihr weiteres Schicksal wurde noch nicht untersucht. Nach gegenwärtigem Wissensstand dürfte es sich bei diesem Mechanismus im wesentlichen um ein reversibles Ereignis handeln. Die Anwendung von Antisense-RNA-Molekülen hat den Vorteil, daß diese Moleküle entweder in vitro synthetisiert und in die Zellen eingebracht werden können oder daß die dafür kodierenden Gene in die Zellen eingeführt werden können, sodaß die inhibierende RNA innerhalb der Zelle produziert werden kann. Es ist jedoch bisher nicht gelungen, solche Gene in eine Form zu bringen, die die Produktion einer wirksamen Menge an Antisense RNA in der Zelle ermöglicht.

In jüngster Zeit wurde ein drittes Prinzip der RNA-Inhibierung entdeckt und der in vitro Anwendung zugänglich gemacht Dieses Prinzip beruht auf der Fähigkeit von RNA-Molekülen, den sog. "Ribozymen", bestimmte RNA-Sequenzen zu erkennen, daran zu binden und sie zu spalten. Es wurde abgeleitet von in den in vivo beobachteten autokatalytischen Spaltreaktionen von RNA-Molekülen in Pflanzenviroiden und Satelliten-RNA.

Aufbauend auf bestimmten Strukturanforderungen für die Ribozym-katalysierte RNA-Spaltung können nunmehr de novo Ribozyme konstruiert werden, die eine auf eine bestimmte Zielsequenz gerichtete in trans Endonukleaseaktivität aufweisen. Da solche Ribozyme, von denen die bisher am eingehendsten erforschte wegen ihrer Struktur "hammer-head"-Ribozyme genannt werden, auf zahlreiche verschiedene Sequenzen wirken können, kann praktisch für jedes beliebige RNA-Substrat das entsprechende Ribozym "maßgeschneidert" werden. Dies macht Ribozyme zu interessanten, äußerst flexiblen Instrumenten für die Inhibierung spezifischer Gene und zu einer vielversprechenden Alternative zu Antisense-Konstrukten, welche bereits einen potentiellen therapeutischen Nutzen gezeigt haben.

Eines der derzeit bekannten, bisher am eingehendsten erforschte Ribozym-Modell umfaßt drei Strukturdomänen; aufbauend auf diesem Modell wurden bereits erfolgreich Ribozyme gegen die CAT-mRNA gebaut (Haseloff et al., 1988; Uhlenbeck et al., 1987):

Die drei Domänen umfassen:
a) eine hochkonservierte Region von Nukleotiden, die die Spaltstelle in 5′-Richtung flankiert. Dabei handelt es sich für gewöhnlich um die Sequenz GUC, wenngleich auch eine Modifikation in GUA oder GUU eine im wesentlichen unverminderte Spaltaktivität zeigte. Spaltung wurde auch nach den Sequenzen CUC, in geringerem Ausmaß auch für AUC und UUC gezeigt (die an die effiziente Spaltung gestellten Anforderungen wurden noch nicht zur Gänze aufgeklärt).
b) die in natürlich vorkommenden Spaltdomänen von Ribozymen enthaltenen hochkonservierten Sequenzen, die eine Art basengepaarten Stiel bilden;
c) die die Spaltstelle auf beiden Seiten flankierenden Regionen, die die exakte Anlagerung des Ribozyms in Relation zur Spaltstelle und um Zusammenhalt von Substrat und Enzym gewährleisten (in den bisher durchgeführten Versuchen wurden beidseitig je 8 Basen gewählt).

RNA-Enzyme können nach diesem Modell konstruiert wurden und haben sich in vitro bereits als für die effiziente und spezifische Spaltung von RNA-Sequenzen als geeignet erwiesen (Haseloff et al., 1988).

In jüngster Zeit wurden weitere Typen von autokatalytischer RNA-Spaltaktivität, die für die zielgerichtete RNA-Inhibierung in Betracht kommen, entdeckt. Eines dieser Modelle ist das sog. "hairpin"-Ribozym, dessen aktive Stelle vom Minus-Strang der Satelliten RNA des Tabak-Ringspotvirus abgeleitet ist (Hampel und Tritz, 1989). Weitere selbst-spaltende RNA-Aktivitäten sind mit dem Hepatitis Delta Virus (Kuo et al., 1988; Sharmeen et al., 1988; Wu et al., 1989) und mit RNAseP (Altmann et al., 1988) assoziiert.

Die den Arbeiten zur vorliegenden Erfindung vorangegangenen Versuche dienten zum Vergleich der Wirkung von Antisense-RNA, Antisense-DNA und Ribozymen. Diese Versuche wurden mit Hilfe der snRNP U7-abhängigen Histon-premRNA-Prozessierungsreaktion durchgeführt, und zwar in einem in vitro System, das U7-abhängig Histon-premRNA prozessiert (Mowry et al., 1987, Soldati et al., 1988). Dabei wurde gefunden, daß Antisense-RNA den stärksten Inhibitor darstellt, wobei die Inhibierung reversibel ist. Die Hemmwirkungen von Antisense-DNA und von Ribozymen vom "hammerhead"-Typ, beide irreversibel, lagen innerhalb derselben Größenordnung, wobei für die vollständige Hemmung jeweils ein ca. 1000 facher Überschuß gegenüber der Substrat-RNA benötigt wurde.

Während die bisherigen Versuche mit Ribozymen mit nackter RNA in proteinfreien Systemen durchgeführt worden waren, waren die Vorversuche zur vorliegenden Erfindung die ersten Experimente, die gezeigt haben, daß synthetisch hergestellte, auf eine spezifische Sequenz gerichtete Ribozyme auch in proteinhaltigem Milieu Spaltwirkung zeigen. Diese Tatsache lieferte einen ersten Hinweis für eine potentielle in vivo Anwendung.

Einer der limitierenden Faktoren bei der Anwendung von Ribozymen zur Inhibierung der Expression von spezifischen Genen dürfte im Aufbau einer Ribozymkonzentration gelegen sein, die ausreicht, die effiziente Ausschaltung einer bestimmten biologischen Reaktion zu bewirken; die Ursachen dafür dürften, ähnlich wie bei der Anwendung von Antisense-RNA, u.a. in der zu geringen Stabilität der RNA gelegen sein.

Der vorliegenden Erfindung lag die Aufgabe zugrunde, ein System für die Anwendung von mRNA als in vivo Inhibitor von mRNA bereitzustellen, das die bisher bestehenden Beschränkungen bei der Anwendung von RNA beseitigt, indem eine wirksame Konzentration an inhibierender RNA in der Zelle zur Verfügung gestellt wird.

Diese Aufgabe wurde erfindungsgemäß durch eine genetische Einheit gelöst, die die für die Transkription durch Polymerase III erforderlichen Transkriptionseinheiten eines tRNA-gens einschließlich der für die Sekundärstruktur der tRNA maßgeblichen Sequenzen enthält und daß die für ein inhibierendes RNA-Molekül kodierenden DNA-Sequenz innerhalb des DNA-Moleküls derart angeordnet ist, daß das inhibieren RNA-Molekül Teil des Transkripts ist.

Bei der Lösung dieser Aufgabe wurde von der Überlegung ausgegangen, daß durch Einführung eines die inhibierende RNA produzierenden Gens gegenüber dem Import der RNA als solchen eine beträchtliche Amplifikation der RNA und damit ein zur Hemmung der biologischen Reaktion ausreichender RNA-Vorrat gewährleistet würde.

Die inhibierende RNA kann ein beliebiges Ribozym oder eine andere mRNA inhibierende RNA, z.B. eine Antisense-RNA, sein.

Grundsätzlich wäre es denkbar, einen.effizienten Transport von RNA bzw. der dafür kodierenden DNA-Sequenz über Viren oder virale Vektoren, z.B. Retroviren, durchzuführen.

Dieses System weist allerdings einige entscheidende Nachteile auf, wie Mobilisierung von endogenen Viren, Rekombination mit endogenen Retroviren, Aktivierung von endogenen Genen durch Integration, Beschränkung hinsichtlich Wirtsorganismus und Gewebsart.

Im Gegensatz dazu wurden daher erfindungsgemäß tRNA codierend Carriergene bereitgestellt, die diese Nachteile nicht aufweisen.

Die als Carriergene für RNA-Gene im Rahmen der vorliegenden Erfindung vorgeschlagenen tRNA-Gene haben folgende Vorteile: sie weisen eine kompakte Struktur auf, können auf Grund ihrer geringen Größe leichter in die Zelle transportiert werden, weisen eine hohe Transkriptionsrate auf und sind in ihrer Expression nicht auf bestimmte Gewebe beschränkt, sondern werden ubiquitär, d.h. in beinahe allen Zelltypen exprimiert.

Ein weiterer Vorteil dieser Polymerase III-Gene liegt im Vorhandensein eines sehr starken Transkriptions-Terminationssignals. Dadurch wird die Wahrscheinlichkeit, daß ein in der Nähe gelegenes zelluläres Gen in unerwünschter Weise aktiviert wird, wesentlich reduziert.

Die von der Polymerase III transkribierten tRNA-Gene haben folgende Eigenarten: es handelt sich dabei um Gene, deren Promotor sich nicht stromaufwärts vor dem Gen, sondern innerhalb des Gens befindet (Geiduschek et al., 1988). Diese für die Bindung der Polymerase III wesentlichen internen Kontrollregionen weisen eine diskontinuierliche Struktur auf; sie bestehen aus zwei sog. "Boxen" (der A-Box und der B-Box), die für die Erkennung durch die Transkriptionsfaktoren wesentlich sind, sowie einem dazwischenliegenden Genabschnitt, der hinsichtlich seiner Länge kritisch ist (Hofstetter et al., 1981). Die Länge dieser Sequenz beträgt bei tRNA-Genen 31-74 Basenpaare (Clarkson).

Vertreter weiterer von Polymerase III transkribierter Gene sind die 5S-RNA und eine Reihe von anderen kleinen Kern- und Zytoplasma-RNA-Genen: 7SK, 7SL, M, U6 und 4,5S RNA, sowie die Adenovirus-Gene VA1 und VA2 (Geiduschek et al., 1988). Gemeinsam ist diesen Genen ihre geringere Größe, ihre kompakte Struktur, ihre hohe Transkriptionsrate und ihre ubiquitäre Transkription.

Es wurde überraschend festgestellt, daß mit Hilfe der erfindungsgemäßen genetischen Einheiten eine erhöhte Stabilität der inhibierenden RNA erzielt werden kann, ohne daß eine Beeinträchtigung ihrer Wirksamkeit im Hinblick auf Aktivität in Kauf genommen werden muß.

Es wurde bereits von Jennings und Molloy, 1987, gezeigt, daß ein spezifischer von Polymerase III erkannter Promotor geeignet ist, die Synthese von Antisense-RNA zu lenken. Dazu wurde das XbaI/BamH1 Fragment des VAl-Gens, enthaltend den Promotor, in 5' Richtung vor die Antisense-DNA kloniert, was dem herkömmlichen Prinzip bei der Verwendung von Polymerase II-Promotoren entspricht. Es wurde angestrebt, ein im Vergleich zu Polymerase II-Transkripten kurzes Transkript zu erhalten. Das Transkript weist nur eine geringfügige Modifikation auf, die eine Basenpaarung der Enden ermöglicht, um die Enden vor Verdau durch Einzelstrang-Exonukleasen zu schützen.

Im Gegensatz zu diesem Vorschlag, in dem nur die Promotorsequenz eines spezifischen Polymerase III-Gens verwendet wird, (der Bereich zwischen Pos.-30 bis 73, während das Wildtyp VA 1 Gen sich bis zur Terminatorsequenz bei Pos.+160 - +200 erstreckt), werden gemäß der vorliegenden Erfindung gezielt zusätzlich diejenigen Sequenzen des Polymerase III-Gens verwendet, die für die Sekundärstruktur des Transkripts maßgeblich sind, um diese für die Stabilisierung der inhibierenden RNA-Sequenzen auszunutzen. Im Gegensatz zum vorbeschriebenen Vorschlag ist die für die inhibierende RNA kodierende Gensequenz innerhalb des von Polymerase III transkribierten Gens so angeordnet, daß erfindungsgemäß eine "Genkassette" vorliegt. Darüberhinaus sind die im Rahmen der vorliegenden Erfindung verwendeten Carrier-Gene im Gegensatz zum viralen VA1-Gen nicht toxisch.

Die von Polymerase III transkribierten tRNA-Gene sind im Rahmen der Erfindung flexibel einsetzbar. Im Hinblick auf ihre Funktion als Carriergene für RNA-inhibisierende Sequenzen sind bei der Auswahl bzw. Modifikation natürlicher bzw. bei der Konstruktion künstlicher Gene folgende Kriterien zu berücksichtigen:
1) die A- und die B-Box sind hoch konserviert.
2) Ein Abschnitt von 5 bis 7 T-Resten stromabwärts der B-Box ist für die Termination der Transkription verantwortlich.
3) Der Abstand zwischen A- und B-Box kann nicht beliebig vergrößert werden, wobei das Maximum derzeit bei etwa 90 bp angenommen wird.
4) In einigen Systemen hat die 5'-flankierende Sequenz einen Einfluß auf die Transkription.
5) Es gibt Hinweise dafür, daß eine intakte Anticodon-Stammregion wesentlich für die Stabilität des Transkripts ist.

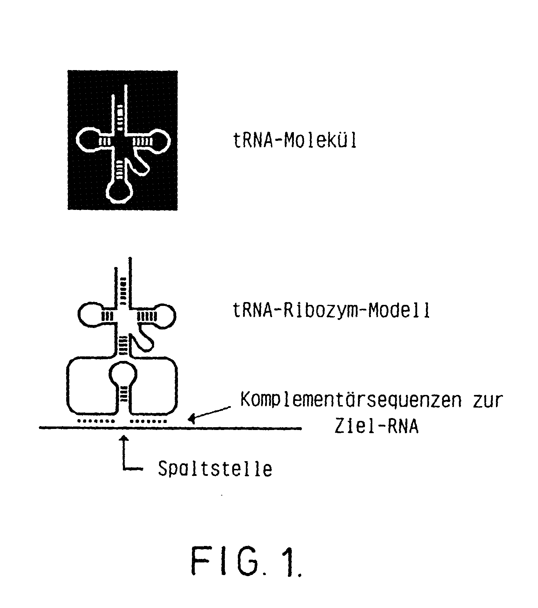

Die von dlen tRNA-Genen kodierten RNAs sind darüberhinaus auf Grund ihrer Kleeblattstruktur als besonders geeignet anzusehen, der inhibierenden RNA Stabilität zu geben. Mit ihrer Hilfe lassen sich kompakte RNA produzierende Geneinheiten herstellen, indem die für die inhibierende RNA kodierende Sequenz zwischen den A- und B-Block eingeführt wird (Fig. 1 zeigt eine schematische Darstellung eines tRNA-Ribozymgens).

Versuche im Rahmen der vorliegenden Erfindung wurden mit der Startmethionin-tRNA durchgeführt, die eine Apa I-Restriktionsstelle in der zwischen A- und B-Block gelegenen Anticodon-Stamm- und Schleifenregion aufweist (die Startmethionin-tRNAs aller höheren Eukaryoten besitzen diese Restriktionstelle in der Anticodon-Schleife).

Neben der zwischen A- und B-Block gelegenen Region sind auch andere Insertionsstellen innerhalb der Carrier-DNA-Sequenzen (tRNA-Gene oder andere Polymerase III-Gene) möglich, solange gewährleistet ist, daß Transkriptionsaktivität der Polymerase III und die Stabilität des Transkripts erhalten bleiben.

Die erfindungsgemäßen genetischen Einheiten können jedoch grundsätzlich mit sämtlichen tRNAs hergestellt werden; erforderlichenfalls kann eine geeignete Restriktionsstelle konstruiert werden, in die dann die für die inhibierende RNA kodierende Sequenz inseriert wird. Bei der Insertion muß lediglich beachtet werden, daß in der Anticodon-Stammregion kein Basenaustausch vorgenommen wird, der die Transkriptionsrate des Gens oder die Stabilität der resultierenden tRNA beeinträchtigt (Folk et al., 1983). Außerdem muß dem Umstand Rechnung getragen werden, daß Inserts größer als 60 bp die Transkription beeinträchtigen können (Ciliberto et al., 1982).

Hinsichtlich der Wahl geeigneter tRNA-Carriergene ist es grundsätzlich erstrebenswert, spezieseigene tRNA-Gene zu verwenden.

Die Versuche im Rahmen der vorliegenden Erfindung wurden durchgeführt, indem Ribozymsequenzen in normale tRNA-Gene eingeführt wurden. Wenn das tRNA-Ribozym, das von diesem Gen transkribiert wird, vorwiegend im Zytoplasma lokalisiert ist, es jedoch für bestimmte Anwendungen, z.B. um kernspezifische RNAs, z.B. solche von snRNP-Partikeln zu inhibieren, wünschenswert ist, das tRNA-Ribozym bzw. die tRNA-Ribozym bzw. die tRNA-Antisense-RNA im Kern zu lokalisieren, können Mutanten verwendet werden, die vorwiegend im Kern lokalisiert sind, z.B. das von Zasloff et al., 1982, und Zasloff, 1983, beschriebene met i tRNA-Gen (Initiations-Met-tRNA-Gen), das einen einzigen Basenaustausch aufweist.

Die erfindungsgemäßen genetischen Einheiten können z.B. wie folgt hergestellt werden:

Eine Kopie des von Polymerase III transkribierten Gens, z.B. eines tRNA-Gens, das auf einem bakteriellen Plasmid enthalten ist, wird mit einem geeigneten Restriktionsenzym an der für die Insertion vorgesehenen Stelle, z.B. zwischen dem A- und dem.B-Block, gespalten und die nach Standardmethoden hergestellte doppelsträngige DNA, kodierend für die inhibierende RNA, hineinligiert. Damit werden geeignete Wirtsorganismen transformiert, selektioniert, vermehrt und die amplifizierte Plasmid-DNA gewonnen. Die Plasmide werden auf das Vorhandensein der erfindungsgemäßen genetischen Einheit überprüft; dies kann durch Restriktionsverdau, Sequenzanalyse oder durch den Nachweis eines funktionellen in vitro Transkripts der Plasmid-DNA erfolgen.

Die so erhaltenen genetischen Einheiten können sowohl in Form des zirkulären Plasmids als auch in Form der aus dem Plasmid herausgeschnittenen Geneinheit, die sämtliche für die Transkription durch Polymerase III erforderlichen Informationen enthält, zur Anwendung gelangen.

Welche Form gewählt wird, richtet sich im allgemeinen nach dem Anwendungsgebiet und nach dem für die Einführung der Geneinheit in die Zelle gewählten Transportsystem.

Die erfindungsgemäßen genetischen Einheiten können auch als Mehrfachkopien vorliegen (dabei handelt es sich um Tandemstrukturen, in denen die genetischen Einheiten mehrfach hintereinander angeordnet sind, wobei die inhibierenden Inserts gleich oder verschieden sein können).

Transkription eines solchen Tandems liefert auf Grund der in jeder einzelnen Einheit enthaltenen Promotorund Terminationssignale voneinander getrennte RNA-Einheiten. Auf Grund dieser Eigenschaft der Fragmente, als jeweils komplette Transkriptionseinheiten vorzuliegen, ist auch die Orientierung der einzelnen Einheiten zueinander irrelevant. Bei der Herstellung solcher Tandems wird von Plasmiden ausgegangen, die multimere Kopien der für die inhibierenden Einheiten kodierenden Region enthalten. Multimere tRNA-Ribozymgene enthaltende Vektoren können z.B. hergestellt werden, indem, ausgehend von der im Beispiel 1 beschriebenen erbBschnitt-Serie, die Einzelfragmente unter Verwendung von T4 DNA-Ligase zu Polymeren ligiert und die polymeren Gene in eine entsprechende Restriktionsstelle eines geeigneten Plasmidvektors rekloniert werden. Die Herstellung solcher Tandems wird durch die geringe Größe der erfindungsgemäßen Einheiten, die vorzugsweise 200 bis 350 bp beträgt, ermöglicht. Mit Hilfe eines solchen "Tandem-Inhibitors" kann nach Herstellung eines heteromeren Komplexes von Antisense-RNAs oder Ribozymen in einem einzigen Versuch die Wirksamkeit eines Satzes von Inhibitoren getestet werden. Z.B. kann auf diese Weise eine Mischung von 5 bis 10 Ribozymen in ein Zellsystem eingeführt werden. Nachdem eine Inhibierung durch die Mischung erfolgt ist, können die einzelnen Ribozyme auf ihre Wirksamkeit untersucht werden. Dies ist insbesondere auf Grund der Tatsache von Vorteil, daß die Kriterien für die Auswahl von zu inhibierenden Ziel-RNA-Sequenzen noch nicht sämtlich erforscht sind.

Als gute Zielsequenzen sind z.B. solche Regionen, die keine Sekundärstruktur aufweisen, Regionen nahe Spaltsignalen, Regionen nach dem Initiationscodon, Regionen ohne Bindungsstellen für spezifische Proteine (wie z.B. Sm-Bindungsstelle in snRNA-Molekülen) anzusehen. Mit Hilfe der vorliegenden Erfindung, insbesondere bei Anwendung in Tandem-Form, wird ermöglicht, Aufschluß über solche Zielsequenzen zu erhalten und sie in der Folge effizient zu inhibieren.

Die Anwendung von Tandem-Einheiten ist auch dann von Vorteil, wenn es für die Inaktivierung von biologischen Prozessen erforderlich ist, die RNA, z.B. virale RNA, an mehreren Stellen zu spalten. Eine Mehrfachspaltung kann z.B. erzielt werden, indem eine größere Anzahl verschiedener Ribozyme konstruiert und ein Vektor, enthaltend die genetischen Einheiten mit den jeweils dafür kodierenden DNA-Sequenzen, in Zellen eingeführt wird. Bei Transkription dieser Sequenzen lagern sich die Ribozymeinheiten an die entsprechenden Zielsequenzen an, wodurch die mRNA in Bruchstücke gespalten wird.

Multimere Kopien können dann verwendet werden, wenn für die Wirksamkeit bei bestimmten Anwendungen, beispielsweise bei der Anwendung des Transferrin-Polykation-Transportsystems, größere Mengen eines niedrigmolekularen Ribozyms erforderlich sind. Plasmide, die multimere Kopien der genetischen Einheit tragen, die die für dieses Ribozym kodierende DNA-Sequenz enthalten, verbessern die Produktion des Fragments durch Erhöhung der Ausbeute um ein Mehrfaches.

Tandem-Einheiten können auch dann vorteilhaft eingesetzt werden, wenn gleichzeitig inhibierende RNAs bereitgestellt werden sollen, die gegen verschiedene RNA-Arten gerichtet sind.

Bei der Anwendung der Erfindung auf Antisense RNA ist zu beachten, daß bei tRNA-Genen die Größe des Inserts beschränkt ist. Im Hinblick auf effiziente Transkription liegt die Größenordnung bei ca. 60 bp; es werden daher bei Verwendung größerer Antisense-RNA-Konstrukte andere von Polymerase III transkribierte Gene, die hinsichtlich der Transkription längerer Sequenzen eine größere Kapazität aufweisen, in Betracht zu ziehen sein.

Die im Rahmen der Erfindung verwendbaren Carriergene können auch synthetisch hergestellt werden, sofern sie die Bedingung erfüllen, die für die Transkription durch Polymerase III erforderlichen Transkriptionseinheiten aufzuweisen. Die Anwendung solcher synthetischen Gene kann folgende Vorteile mit sich bringen:
a) Solche Gene werden von Aminoacylsynthetasen und von Ribosomen nicht erkannt, wodurch ein Eingriff in die Translationsmaschinerie der Zelle verhindert wird.
b) Es besteht die Möglichkeit, durch die Schaffung synthetischer Konstrukte inhibierende RNAs mit größerer Stabilität und höherer Transkriptionsrate herzustellen als mit einem natürlichen Gen.
c) Der Klonierungsprozeß kann durch Schaffung synthetischer Sequenzen flexibler gestaltet werden.

Es wurde im Rahmen der vorliegenden Erfindung festgestellt, daß die Stabilität der Ribozym-tRNA-Moleküle erhöht werden kann, indem die Anticodon-Stammregion des Carrier-tDNA-Moleküls verlängert wird. Es konnte gezeigt werden, daß durch Verlängerung der 5 Basenpaare aufweisenden Anticodon-Stamm-Region des Wildtyp-tRNA-Gens auf 9 Basenpaare eine gegenüber dem verkürzten Wildtyp tRNA-Gen um das 6-fache erhöhte Menge an Transkript erzielbar ist, was auf eine Erhöhung der Stabilität und die damit erzielbare Prozessierbarkeit zurückgeführt werden kann.

Es wurde weiters festgestellt, daß eine Erhöhung der Stabilität der erfindungsgemäßen genetischen Einheiten in Form von tRNA-Ribozym-Genen oder tRNA-Antisense-Genen auch erreicht werden kann, wenn die für die inhibierende RNA-Funktion kodierenden DNA-Sequenzen als Teil von Introns vorliegen (im Fall von Ribozymen als "Ribintrons" bezeichnet). Dabei wurde von der Überlegung ausgegangen, daß natürlich vorkommende Introns die Sekundärstruktur von tRNA-Precursormolekülen nicht verändern und die tRNA-Introns keine Sequenzkonservierung zeigen, wodurch durch Klonierung von Ribozym- oder Antisense-Sequenzen als Teil von Introns die Strukturveränderung des resultierenden tRNA-Precursors minimiert und dessen Stabilität damit maximiert werden können. An Hand des im Rahmen der vorliegenden Erfindung verwendeten tyrtRNA-Gens, bei dem das Spleißen der tRNA-Precursormoleküle nur langsam erfolgt, konnte gezeigt werden, daß durch die Verwendung von "Ribintron"-Sequenzen enthaltenden tDNA-Molekülen die Aktivität von ribtRNA wirkungsvoll erhöht werden kann. Damit ist es möglich, verstärkt im Zytoplasma lokalisierte RNAs anzugreifen. Dieses System ermöglicht auf einfache Weise den Einbau geeigneter Strukturelemente zur Erhöhung der Stabilität der "Ribintrons" gegenüber dem Abbau durch Exonukleasen. Dies kann z.B. durch zusätzliche Basenpaarungen oder durch größere Hairpin-Bereiche angrenzend an die Ribozymsequenzen erzielt werden. Bei derartigen Modifikationen ist zu beachten, daß die für das Splicing des Introns maßgeblichen Strukturen erhalten bleiben.

Die natürlichen Intronsequenzen können modifiziert werden, z.B. durch Einfügen geeigneter Restriktionsschnittstellen, um die Klonierung von Oligonukleotiden zu ermöglichen, oder, soweit nicht im natürlich vorkommenden Gen enthalten, durch Einfügen von Nukleotiden, die eine Basenpaarung mit dem Anticodon-Triplett ermöglichen, um über ein zusätzlich stabilisierendes Strukturmerkmal zu verfügen.

Es konnte im Rahmen der vorliegenden Erfindung gezeigt werden, daß die Expression von Ribozymen als Bestandteil von Introns im wesentlichen keine Beeinträchtigung der tRNA-Sekundärstruktur mit sich bringt, wodurch das entstehende Transkript in hoher Konzentration akkumuliert und korrekt prozessiert werden kann. Falls sich das während des Prozessierens freigesetzte Intron als nicht genügend stabil erweist, können geeignete Strukturmerkmale vorgesehen werden, die eine Erhöhung der Stabilität gegenüber Exonukleaseabbau bewirken.

Um die erfindungsgemäßen genetischen Einheiten in die Zelle einzubringen, sind mehrere Methoden anwendbar:

Die Standardmethode zum Einführen von DNA in Gewebekulturzellen benutzt die Bildung eines Co-Präzipitats zwischen der DNA und Calciumphosphat (Graham et al., 1973). Das Präzipitat wird Zellen beigegeben, die einen bestimmten Anteil aufnehmen, möglicherweise durch einen Pynozytose-Vorgang. Eine ähnliche Methode benutzt ein positiv geladenes Material, DEAE-Dextran, das die Aufnahme von DNA durch die Zelle erleichtert. Es wurden auch Methoden entwickelt, DNA durch Elektroporation (dabei entstehen durch ein pulsierendes elektrisches Feld vorübergehend Poren) in die Zelle zu bringen (Langridge et al., 1987, Fromm et al., 1987). Mikroinjektionstechniken für die Einführung in große Zellen (Kressmann et al., 1980) und Gewebekulturzellen (Pepperkok et al., 1988) sind ebenfalls anwendbar. Diese Methoden sind jedoch nur im Labor bzw. für in vitro Anwendungen brauchbar. Kürzlich wurde ein synthetisches kationisches Peptid (DOTMA) entwickelt, das spontan mit DNA Liposomen bildet und so den Transport in die Zellen erleichtert (Felgner et al., 1987). Grundsätzlich sind, wie bereits erwähnt, auch retrovirale Vektoren für den Transfer von genetischen Material in die Zelle geeignet (Stewart et al., 1986, 1987); diese Systeme weisen jedoch die bereits angeführten Nachteile auf.

Ein weiterer Transportmechanismus beruht auf der Benutzung "entwaffneter" Toxine als Transportvehikel.

Die bisher verwendeten Transportverfahren sind sämtlich von dem Mangel behaftet, nicht genügend inhibierende Nukleinsäure in die Zelle befördern zu können. Mit Hilfe der vorliegenden Erfindung ist es nunmehr aufgrund der geringen Größe und der Kompaktheit der Moleküle möglich, bezogen auf die Transportkapazität eine Steigerung der Zahl an aktiven Inhibitoreinheiten zu erzielen. Aufgrund der geringen Größe und kompakten Struktur der erfindungsgemäßen genetischen Einheiten kann nach geringfügigen Modifikationen, z.B. Konjugation mit Cholesterin, lipophilen Gegenionen oder Kernlokalisierungspeptiden auch auf ein Transportsystem zur Gänze verzichtet werden.

Bevorzugt wird im Rahmen der vorliegenden Erfindung ein lösliches System für den Transport angewendet, das über Rezeptor-vermittelte Endozytose abläuft. Besonders bevorzugt wird dabei ein Transferrin-Polykation-Konjugat mit der erfindungsgemäßen genetischen Einheit komplexiert; der Komplex wird durch den Transferrin-Rezeptor, der auf praktisch allen wachsenden Zellen vorhanden ist, aufgenommen.

Die Anwendungsgebiete für die vorliegende Erfindung sind zahlreich: z.B. können transgene Tiere hergestellt werden, die auf Grund des Vorhandenseins der erfindungsgemäßen genetischen Einheiten im genetischen Material eine intrazelluläre Immunität gegen Viren, z.B. Maul- und Klauenseuchevirus, Newcastle Disease Virus, Rinderpapillomavirus, Pseudorabies oder infektiöse Gastroenteritis, aufweisen. Entsprechend kann auch in transgenen Pflanzen intrazelluläre Immunität, z.B. gegen das Kartoffelvirus PVX, erzeugt werden.
Weiters können die erfindungsgemäßen genetischen Einheiten in somatische Zellen eingebracht werden, um gegen pathogene Viren, wie HIV oder verwandte Retroviren gerichtete Ribozyme bzw. Antisense-RNAs zur Bekämpfung dieser viralen Erreger einzusetzen.

Ein weiteres Anwendungsgebiet liegt in der Gentherapie durch Anwendung von RNA Konstrukten mit Komplementarität zu Oncogenen oder anderen Schlüsselgenen, die Wachstum und/oder Differenzierung von Zellen kontrollieren. Bei derartigen Anwendungen kommt die mit Hilfe der vorliegenden Erfindung wirksam erzielbare hohe Spezifität der RNA-Inhibierung, mit Hilfe derer z.B. eine Unterscheidung zwischen Protooncogen- und Oncogentranskripten möglich ist, zum Tragen.

Weiters können die erfindungsgemäßen genetischen Einheiten verwendet werden, um in Pflanzen oder Tieren die Expression von spezifischen Genen zu verhindern, um auf diese Weise erwünschte Eigenschaften hervorzubringen.

Die inhibierende Wirkung von RNA kann auch bei der Bekämpfung von Krankheiten derart ausgenutzt werden, daß die Produktion von unerwünschten Genprodukten unterbunden wird, z.B. die Produktion des bei der Alzheimer-Krankheit auftretenden Haupt-Plaqueproteins ("major plaque protein") oder von Autoimmunerkrankungen verursachenden Proteinen.

Die vorliegende Erfindung kann auch in denjenigen Fällen angewendet werden, in denen ein regulatorisches Protein, das eine Wechselwirkung mit RNA aufweist, durch Anlagerung von RNA ausgeschaltet werden soll.

Pharmazeutische Zubereitungen, die die erfindungsgemäßen genetischen Einheiten als wirksame Komponente enthalten, etwa in Form von Lyophilisaten, sind ebenfalls Gegenstand der Erfindung. Ihre Anwendung umfaßt die angegebenen Indikationsgebiete.

Anhand der im Rahmen der vorliegenden Erfindung durchgeführten Versuche konnte Transkriptionsaktivität des tDNA-Ribozymgens nachgewiesen werden. Dazu wurde ein tDNA-Ribozym-Genkonstrukt hergestellt, indem eine für ein 53 bp langes, gegen die snRNA U7 Sequenz gerichtetes Ribozym kodierende DNA-Sequenz in die Apal Restriktionsstelle zwischen die A-Box und die B-Box der Start-Methionin-tDNA inseriert wurde (die A- und die B-Box sind die beiden Erkennungssequenzen für die Polymerase; die Transkription beginnt 15 bp stromaufwärts der A-Box und endet an einer Oligo T-Sequenz stromabwärts der B-Box). Nach Mikroinjektion dieses Gens wurde Transkription nachgewiesen; die Konzentration des tRNA/Ribozym-Hybrids betrug 10-20 % der Konzentration an tRNA, die von einem co-injizierten Wildtyp-tRNA-Gen produziert wurde.

RNA-Moleküle, synthetisiert in vitro vom tRNA-Ribozymgen, spalten die Ziel-RNA an der vorgesehenen Stelle. Das Hinzufügen der tRNA-Struktur zur Ribozymsequenz blockiert nicht die Ribozymaktivität. tRNA-Ribozymmoleküle, synthetisiert von Genen, die in Oozyten injiziert worden sind, spalten die Ziel-RNA ebenfalls an der vorgesehenen Stelle und mit derselben Effektivität wie in vitro synthetisierte Ribozyme ohne die zusätzliche tRNA-Struktur. Das beweist, daß in vivo Synthese und Prozessierung eines tRNA-Ribozyms nicht mit Modifikationen einhergehen, die die Wirkung des Ribozyms behindern.

Im Rahmen der vorliegenden Erfindung wurde erstmals in vivo die Aktivität eines Ribozyms nachgewiesen. Dazu wurden tDNA/Ribozym-Gene zusammen mit radioaktiv markiertem GTP in den Kern von Oozyten injiziert. Nach achtstündiger Inkubation, die für die Ribozymsynthese vorgesehen war, wurde die radioaktiv markierte Substrat-RNA (U7 RNA) ins Zytoplasma der Oozyten injiziert. Nach weiteren zwei Stunden wurde die Nukleinsäure aus den Oozyten präpariert: In den Oozyten, in denen Ribozymsynthese stattgefunden hatte, wurde keine verbleibende Substrat-RNA nachgewiesen. Im Gegensatz dazu war die Substrat-RNA in denjenigen Oozyten stabil, die nicht mit dem tDNA/Ribozymgen injiziert worden waren bzw. bei denen das Gen den Kern verfehlt hatte.

Es konnte im Rahmen der vorliegenden Erfindung auch gezeigt werden, daß die erfindungsgemäßen tDNA-Ribozyme die transformierende Wirkung eines Onkogens inhibieren können. Anhand von erythroiden Hühnerzellen, transformiert mit dem erbB-Onkogen, wurde die Aktivität eines tRNA-Ribozyms über die durch Inhibierung der erbB Expression eintretende Differenzierung der Zellen in Erythrozyten nachgewiesen.

Die Wirksamkeit der erfindungsgemäßen genetischen Einheiten kann auch durch Beobachtung der Resistenz von Mauszellen gegen Infektionen, beispielsweise Polyomainfektionen, nach Anwendung von erfindungsgemäßen genetischen Einheiten, die gegen das Virus (gegebenenfalls gegen mehrere Regionen), z.B. gegen das Papilloma-Virus gerichtet sind, überprüft werden.

### Beispiel 1

### Konstruktion von tRNA Ribozymgenen

### a) Konstruktion von pSPT18met1

Das Methionin-Initiator 1-tRNA-Gen von Xenopus, vorhanden auf einem 284 bp EcoRI-Fragment, das in pBR322 kloniert war (das HinfI H-G-Fragment (Hofstetter et al., 1981, Tellford et al. 1979), wurde durch EcoRI Verdau des pBR322 Vektors isoliert, durch Gelelektrophorese (2 % Agarose/TBE) gereinigt und in die EcoR1 Stelle des bakteriellen Plasmids pSPT18 (Boehringer Mannheim) so ligiert, daß bei Transkription des Plasmids mit SP6-Polymerase ein Sense-tRNA-Transkript erhalten wurde. Dazu wurden Standardklonierungsmethoden, beschrieben in (Maniatis), verwendet. Der Hauptvorteil der Reklonierung des tRNA-Gens in pSPT18 liegt im Vorhandensein von einander gegenüberliegenden SP6- und T7-RNA Polymerase-Promotoren auf dem Plasmid. Daher können durch in vitro Transkription spezifische RNA-Transkripte, die entweder die tRNA-Ribozymsequenz oder die dazu komplementäre Sequenz enthalten, erhalten werden (Melton et al., 1984). Diese Transkripte sind nützlich, um die Spaltaktivität des RNA-Moleküls zu testen oder um durch ein "RNase Protection Mapping" die Anwesenheit von tRNA-Ribozymen in Zellextrakten, die das tRNA-Ribozym exprimieren, nachzuweisen.

### b) Konstruktion von tRNA-Ribozymgenen

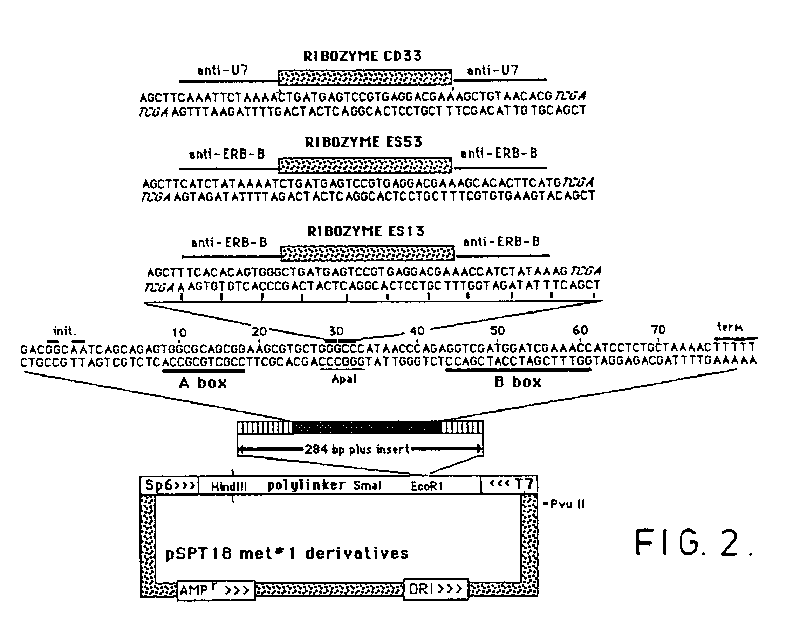

Das tRNA-Gen auf pSPT18met1 wurde an der einzigen ApaI Stelle in der Anticodon-Stamm- und Schleifenregion gespalten (s. Fig. 2). Diese Abbildung zeigt Plasmide, die die für tRNA-Ribozym-kodierende Sequenzen enthalten. pSPT18 met1 enthält das 284 bp EcoR1-Fragment, das das Xenopus laevis Initiator-tRNA-Gen trägt. Dabei handelt es sich um das G-H Fragment (Hofstetter et al., 1981), kloniert in die EcoR1-Stelle des Polylinkers von pSPT18. Die komplementären Oligonukleotide, kodierend für Ribozyme, die gegen U7snRNA (CD33 und die beiden Sequenzen der erbB-mRNA (ES13, ES53) gerichtet sind, werden gezeigt. Die hier verwendete Klonierungsstrategie ergab die Entfernung der überstehenden Enden der ApaI-Stelle im tRNA-Gen. Der Anteil des Inserts, kodierend für das Ribozym und die zur Ziel-RNA komplementären Regionen (Anti-U7, Anti-erbB) ist gekennzeichnet, ebenso wie die A- und B-Box, der Abschnitt der 5 T-Reste (Terminationssignal) und die Transkriptionsinitiationsstellen. Das Plasmid enthält den ColEI Replikationsursprung, einen Ampicillinresistenz-Marker und die Promotoren für T7-und SP6-RNA-Polymerase.

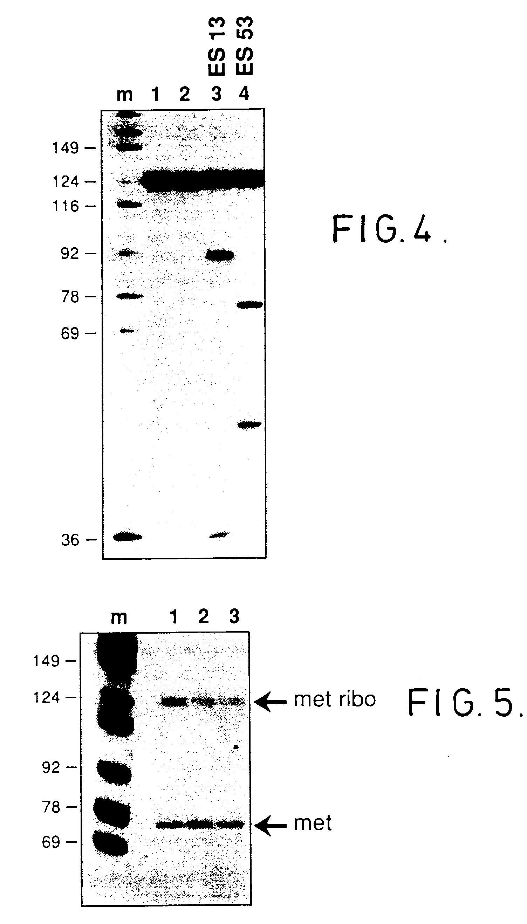

Zur Herstellung doppelsträngiger synthetischer DNA-Oligonukleotide, kodierend für die viroide Spaltsequenz (Haseloff et al., 1988) flankiert von den zur Ziel-mRNA komplementären Sequenzen, wurden zunächst einzelsträngige Oligonukleotide nach Standardmethoden (Applied Biosystems DNA synthesizer) hergestellt. Komplementäre Oligonukleotide wurden phosphoryliert, annealt und in das ApaI gespaltene pSPT18met1 Plasmid unter Verwendung von Standardmethoden hineinligiert (Maniatis). Die Ligationsmischung wurde zur Transformation von E.coli HP101 verwendet, Bakterienklone, die das neue Plasmid enthielten, wurden isoliert und das Vorhandensein von aktiven Ribozymsequenzen auf dem bakteriellen Plasmid auf zwei Arten bestätigt:
1) RNA-Moleküle, die von der in vitro SP6-Transkription klonierter DNA-Plasmide stammten, wurden mit einer radioaktiv markierten RNA, enthaltend die Zielsequenz für das Ribozym, inkubiert und auf spezifische Spaltung der Ziel-RNA getestet (s. Fig. 4).
2) Das Vorhandensein korrekt inserierter DNA-Sequenzen wurde durch Dideoxy-DNA-Sequenzierung über die Insertionsstelle bestätigt.

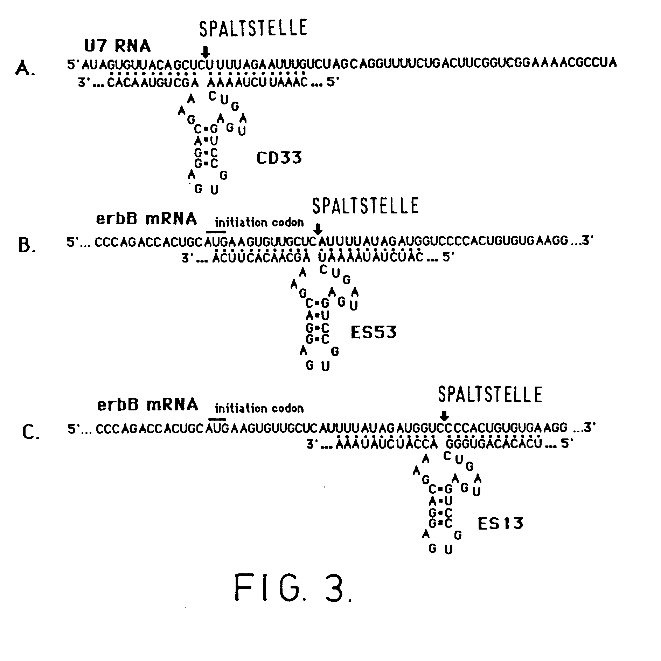

Fig. 4 zeigt die in vitro Ribozymaktivität der tRNA-Ribozyme. Plasmid-DNA-Moleküle, die die erbBschnitt-tRNA-Ribozyme ES13 und ES53 trugen, wurden mit PvuII verdaut und mit SP6-RNA-Polymerase transkribiert. Diese Transkription lieferte 230 Nukleotide lange RNA-Moleküle, die die tRNA-Ribozymsequenz und zusätzlich die 5′- und 3′flankierenden Sequenzen, die von den flankierenden Xenopussequenzen und den flankierenden bakteriellen Plasmidsequenzen stammen, enthalten (s. Fig. 2). Die Ribozymtranskripte wurden mit 20.000 cpm (20 fM) eines RNA-Moleküls mit der Region der erbB-mRNA, umfassend das Initiationscodon, inkubiert. Das RNA-Molekül besitzt die Zielsequenzen sowohl für ES13 und ES53 (s. Fig. 3). Nach 2stündiger Inkubation des Ribozyms plus Ziel-RNA bei 37°C in Gegenwart von 10 mM MgCl₂, 20 mM TRIS-HCl, pH 7,5 und 150 mM NaCl, wurde EDTA zu einer Konzentration von 15 mM zugefügt, die Probe getrocknet, in 80% Formamid/TBE gelöst, 30 sek. bei 95°C erhitzt und auf einem 9,5% Acrylamid/8,3 M Harnstoff/ TBE-Gel aufgetrennt. Nach der Elektrophorese wurden die markierten RNA-Moleküle durch Autoradiographie nachgewiesen.

Spur M: Molekulargewichtsmarker: pBR322 DNA, gespalten mit HpaII und unter Verwendung des Klenow-Fragments der DNA-Polymerase mit alpha-³²P-CTP radioaktiv markiert (Maniatis). Die Molekulargewichtsmarker wurden unmittelbar vor dem Auftragen auf das Gel in 80% Formamid/TBE gelöst und 3 min. bei 95°C erhitzt. Die Molekulargewichte einiger der Fragmente (in Nukleotiden) sind links angegeben.
Spur 1: erbB-Ziel-mRNA (20.000 cpm, 20 fM) ohne Inkubation.
Spur 2: erbB-Ziel-mRNA (20.000 cpm, 20 fM), inkubiert mit MgCl₂ bei 37°C ohne Ribozyme.
Spur 3: erbB-Ziel-mRNA (20.000 cpm, 20 fM), inkubiert mit ES13-RNA (1 fM).
Spur 4: erbB-Ziel-mRNA (20.000 cpm, 20 fM), inkubiert mit ES53-RNA.

Fig. 3 zeigt die Komplementarität zwischen Ribozymen und Ziel-RNAs: CD33 (A) gegen U7snRNA Cotten et al., 1988), ES13 (C) und ES53 (B) gegen Sequenzen der erbBmRNA (Venustrom et al., 1980).

Der tRNA-Anteil der tRNA-Ribozyme ist aus Gründen der Übersichtlichkeit nicht gezeigt. Die Spaltstellen für das Ribozym sind gekennzeichnet, ebenso die Initiationscodons der erbB-mRNA.

### Beispiel 2

### tRNA-Ribozymtranskription in Xenopus Oozyten.

Die Transkription der tRNA-Ribozymgene, mikroinjiziert in Xenopus-Oozyten, wurde nach der in (Kressmann et al., 1978, Hofstetter et al., 1981, Kressmann et al. 1980) für tDNA beschriebenen Methode durchgeführt. Dazu wurde wie folgt vorgegangen: Stadium VI Oozyten wurden erhalten von HCG (Humanes Chorion Gonadotropin)-stimulierten erwachsenen Xenopus laevis Weibchen. Die Oozyten wurden kurz zentrifugiert, um den Kern an den Rand der Oozyte zu bringen. Jeder Kern wurde mit ca. 50 nl einer Lösung, enthaltend 0,3 µg/µl supercoiled Plasmid DNA (enthaltend das tRNA-Ribozymgen gemäß Beispiel 1 ) und 2 µCi/µl ³²P-GTP injiziert. Nach 5 bis 8 stündiger Inkubation bei 20°C wurden die einzelnen injizierten Oozyten in 1% SDS, 1 mg/ml Proteinase K, 300 mM NaCl, 20 mM Tris, pH 8, 20 mM EDTA (400 µl/Oozyte) bei 37°C 45 min. verdaut, einmal mit Phenol und einmal mit Phenol/Chloroform extrahiert und mit Ethanol gefällt. Die gesammelten Ethanol-Präzipitate wurden in 80% Formamid/TBE gelöst, kurz zwecks Denaturierung bei 95°C erhitzt und durch Elektrophorese auf einem 10% Acrylamid/8,3 M Harnstoff/TBE-Gel aufgetrennt und durch Autoradiographie sichtbar gemacht. Bei allen Versuchen mit tRNA-Ribozymgenen enhielten die Injektionslösungen das Wildtyp metRNA Gen mit einer Konzentration von 1/6 der Konzentration des tRNA-Ribozymgens. TBE-Puffer (Tris, Borat, EDTA) wurde nach der Vorschrift, beschrieben in (Maniatis), hergestellt.

### Das Ergebnis dieser Versuche zeigt Fig. 5:

Spur m: Molekulargewichtsmarker: wie in Fig. 4. Die Molekulargewichte einiger der Fragmente (in Nukleotiden) sind links in der Fig. angegeben. Spuren 1,2,3: Die Nukleinsäure von einzelnen Oozyten, injiziert mit dem Met-tRNA-Gen und dem Met-tRNA-Ribozymgen metribo 33. Rechts in der Figur sind die Positionen der Met-tRNA (met,77 Nukleotide lang) und des tRNA-Ribozyms (met ribo), 128 Nukleotide lang) angegeben.

### Beispiel 3

Bestimmung der Ribozymaktivität von Oozyten synthetisiertem tRNA-Ribozym im Vergleich mit in vitro synthetisiertem Ribozym, das keine tRNA-Sequenzen aufweist.

Ein gegen U7 gerichtetes tRNA-Ribozym, synthetisiert in mikroinjizierten Oozyten wurde durch Auftrennung mittels Elektrophorese erhalten, durch Autoradiographie sichtbar gemacht, aus dem Polyacrylamidgel herausgeschnitten und durch Inkubation über Nacht in einem Eppendorf Vibrator in HEP ("Heidelberg Extraktionspuffer": 0,75 M Ammonium-acetat, 10 mM Magnesiumacetat, 1 % (Vol./Vol.) Phenol, 0,1 % (Gew./Vol.) SDS, 0,1 mM EDTA) eluiert. Die eluierte RNA wurde einmal mit Phenol/Chloroform und einmal mit Chloroform extrahiert und mit Ethanol in Gegenwart von 10 µg E.coli tRNA als Träger gefällt. Das Präzipitat wurde aufgenommen und mittels Cerenkov-Zählung der ³²P-Markierung unter Verwendung der Werte für die spezifische Aktivität (Kressmann et al., 1982) quantitativ bestimmt. Proben des tRNA-Ribozyms wurden mit ³²P-markierter RNA, enthaltend die U7-Sequenz (10.000 cpm/Probe, 10 fM plus die angegebenen Mengen an unmarkierter U7-RNA) 2h bei 37°C in Anwesenheit von 150 mM NaCl, 10 mM MgCl₂ und 20 mM Tris-HCl, pH 7,5 inkubiert. Die Reaktionen wurden durch Zusatz von EDTA auf 15 mM gestoppt, die Proben wurden getrocknet, in 80% Formamid/TBE gelöst, bei 95°C 30 sek. erhitzt und auf einem vorgewärmten 9,5 % Acrylamid/ 8,3 M Harnstoff/ TBE-Gel aufgetrennt. Die radioaktiv markierten RNA-Arten wurden durch Autoradiographie bei -80°C mit einer Dr. Goos "special" Verstärkerfolie sichtbar gemacht.

Das Ribozym CD32 wurde erhalten durch T7-Polymerase-Transkription eines Plasmids, enthaltend das Insert von CD33 (s. Fig. 2) und kloniert in die Hind III/Sal I-Stellen von pSPT19 (Boehringer Mannheim). Dieses Transkript enthält nur die Ribozymsequenz plus die zu U7 komplementären Sequenzen, flankiert von kurzen Abschnitten der Vektorsequenz. Die Spaltaktivität dieses Ribozyms wurde als Vergleich mit der Spaltaktivität des oozytensynthetisierten tRNA-Ribozyms CD 33 herangezogen, um den Einfluß der Sekundärstruktur der tRNA sowie der in vivo Synthese und -Modifikation auf die Ribozymaktivität zu beurteilen. Es zeigte sich, daß beide Ribozyme (CD 32 und CD 33) die U7-Sequenz enthaltende RNA (94 Nukleotide lang) in ein 5′-Spaltprodukt von 25 Nukleotiden und ein 3′-Spaltprodukt von 69 Nukleotiden spalten.

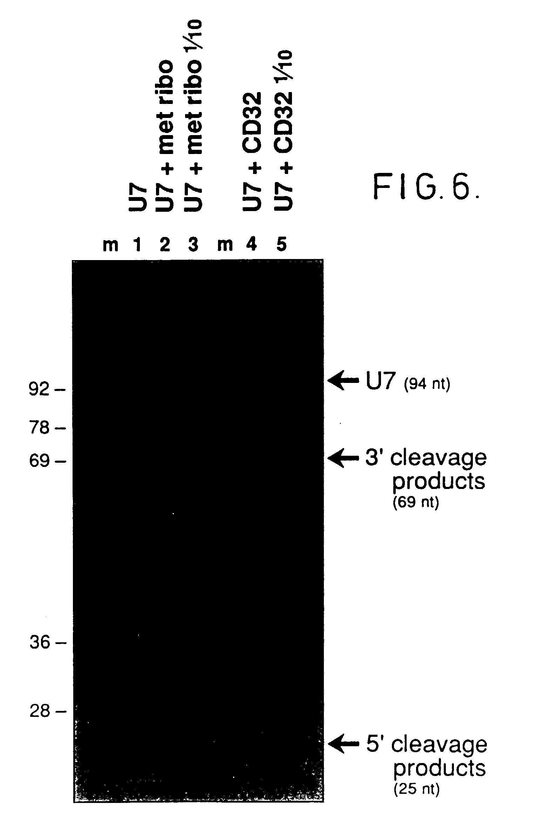

Das Ergebnis dieser Versuche zeigt Fig. 6:
Spuren m: Molekulargewichtsmarker analog Fig. 5
Spur 1: U7-RNA (10.000 cpm, 10 fM), inkubiert ohne Ribozym.
Spur 2: U7-RNA (10.000 cpm, 100 fM), inkubiert mit 10 fM des oozyten-synthetisierten tRNA-Ribozyms CD33
Spur 3: U7-RNA (10.000 cpm, 1 pM), inkubiert mit 10 fM oozyten-synthetisiertem tRNA-Ribozym CD33.
Spur 4: U7-RNA (10.000 cpm, 100 fM), inkubiert mit 10 fM von in vitro mit T7-Polymerase synthetisiertem Ribozym CD32.
Spur 5: U7-RNA (10.000 cpm, 100 fM), inkubiert mit 1 fM von in vitro mit T7-Polymerase synthetisiertem Ribozym CD32.

### Beispiel 4:

### Bestimmung der Spaltung von Ribozymsubstrat in Oozyten

Eine Mischung von ³²P-GTP, Anti U7-tRNA-Ribozymgen und das Met-tRNA-Gen wurden in Oozytenkerne injiziert. Die injizierten Oozyten wurden, wie in Beispiel 2 beschrieben, bei 20°C 8 h inkubiert, um Transkription stattfinden zu lassen. Daraufhin wurde radioaktiv markierte U7-RNA (50 nl, 100.000 cpm/µl, 100 fM/µl) ins Cytoplasma der Oozyten injiziert. Die Oozyten wurden daraufhin 2 h lang inkubiert. Die Präparation der Nukleinsäuren von einzelnen Oozyten und ihre Auftrennung mittels Gelelektrophorese wurden durchgeführt wie in oben beschrieben.

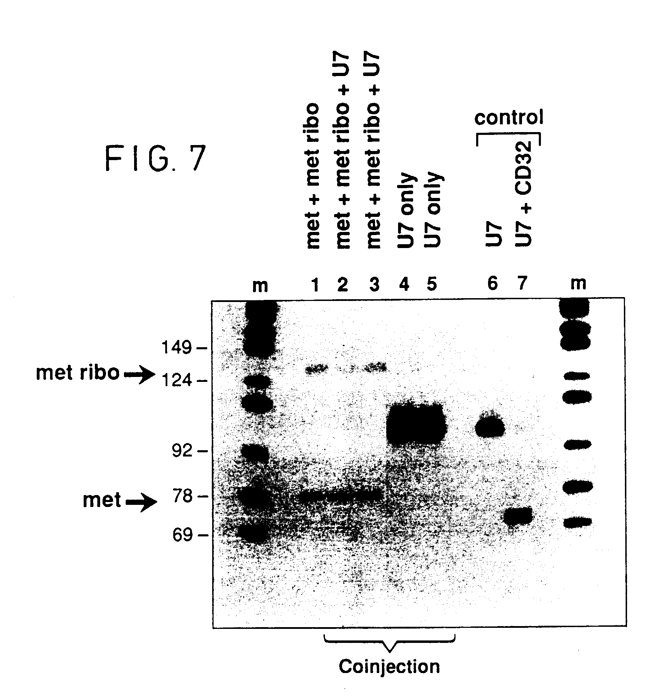

Das Ergebnis dieser Versuche zeigt Fig. 7:
Spuren m: Molekulargewichtsmarker wie Fig. 5
Spur 1: Nukleinsäuren aus einer Oozyte injiziert mit den Met- und Metribo-Genen
Spuren 2 und 3: Oozyten, injiziert mit Met und Metribo, gefolgt von U7-RNA-Injektion.
Spuren 4 und 5: Oozyten,nur mit U7-RNA injiziert.
Spur 6: Ein Aliquot der U7-RNA verwendet für die Injektion.
Spur 7: U7-RNA (10 fM), inkubiert mit dem Ribozym CD32 (10 fM) während 2h bei 37°C in Gegenwart von 150 mM NaCl, 10 mM MgCl₂ und 20 mM Tris-HCl, pH 7,5. Wegen der Gelbedingungen ist nur das 3′-Spaltprodukt (69 Nukleotide) gezeigt.

### Beispiel 5:

### Transkriptionsaktivität von tRNA-Ribozymen in Hühnerzellen

Plasmid-DNA-Moleküle, enthaltend die tRNA-Ribozymgene wurden in Hühnerzellen eingeführt, um die Transkriptionsaktivität der Gene zu bestimmen. Es wurde gezeigt, daß das tRNA-Ribozymgen (abgeleitet von einem Xenopus-tRNA-Gen) in Hühnerzellen effizient transkribiert wird. Dazu wurde wie folgt vorgegangen: 10⁶ primäre Hühnerembryo-Fibroblastenzellen (Zenke et al., 1988) wurden pro 10 cm-Schale nach Standardmethode ausgesät und über Nacht wachsen gelassen. Am Morgen wurde jede Schale mit 10 µg Plasmid-DNA (enthaltend entweder erbBschnitt 13 oder eberbBrBschnitt 53) unter Verwendung der Calciumphosphat-Copräzipitations-Methode (Graham et al., 1973) transfiziert. Die Zellen wurden über Nacht dem Präzipitat ausgesetzt, am darauffolgenden Morgen zweimal mit frischen Medium gewaschen und weitere 48 h in frischen Medium inkubiert. Das Medium wurde daraufhin entfernt, die Zellen in PK/SDS-Puffer (s. oben) aufgenommen und die Nukleinsäure gewonnen. Daraufhin wurde die Nukleinsäure einem "RNAse Protection Mapping" unter Verwendung von ³²P-markierten Antisense erbBschnitt 13 oder erbBschnitt 53 RNA-Sonden unterzogen, um die Anwesenheit von tRNA-Ribozym-Transkripten nachzuweisen. Markierte RNA (10.000 cpm/ 10 fM Antisense-RNA pro Probe) wurde dem getrockneten Ethanolpräzipitat zugefügt, die Probe abermals getrocknet und in 10 µl 80% entionisiertem Formamid, 400 mM NaCl, 20 mM PIPES, pH 6,5, 10 mM EDTA gelöst. Die Proben wurden mit sterilem Paraffinöl überschichtet, 3 min. bei 95°C erhitzt und rasch in ein 45°C Wasserbad überführt, in dem über Nacht bebrütet wurde. Am Morgen wurden 0,3 ml eiskaltes NaCl (300 mM), 30 mM Tris, pH 7,5, 1 mM EDTA, 0,05 mg/ml RNase A und 80 Einheiten/ml RNase T1, unter rascher, vorsichtiger Bewegung zugefügt. Die Proben wurden bei Raumtemperatur 45 min. bebrütet. Proteinase K und SDS wurden auf 1 mg/ml und 0,5 % zugefügt, die Inkubation bei Raumtemperatur und anschließend bei 56°C je 20 min. fortgesetzt. Die Probe wurde nach Zusatz von 10 µg tRNA mit Ethanol gefällt. Der aufgenommene Niederschlag wurde in 80% Formamid/TBE aufgenommen und auf einem vorgewärmten 9,5 % Acrylamid/8,3 M Harnstoff/TBE-Gel aufgetrennt.

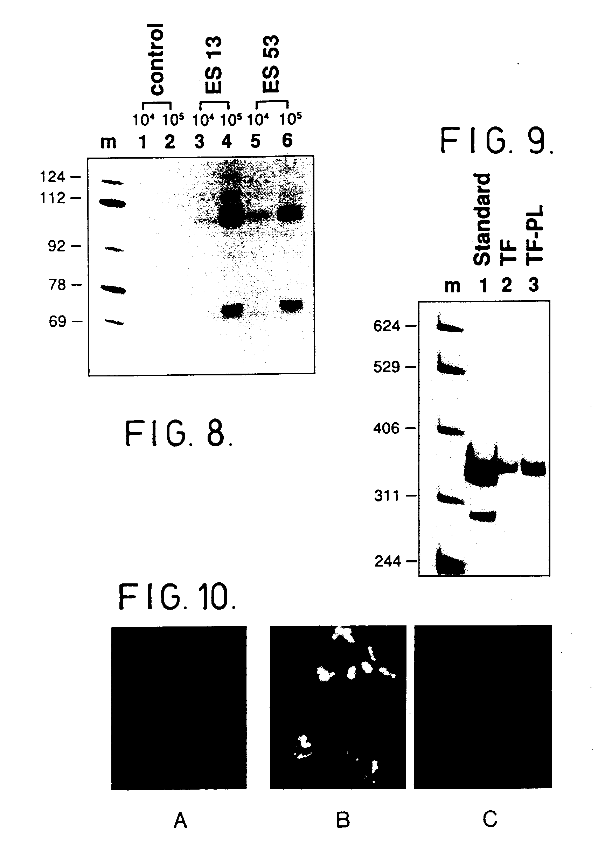

### Das Ergebnis dieses Versuches zeigt Fig. 8:

Spuren m: Molekulargewichtsmarker wie in den vorangegangenen Beispielen
Spuren 1 und 2: Mapping der Nukleinsäuren von 10 000 und 100 000 Zellen, die nicht mit Plasmid DNA, hybridisiert mit der ES 13 Sonde, transfiziert waren
Spuren 3 und 4: Mapping der Nukleinsäuren von 10 000 und 100 000 Zellen, transfiziert mit ES 13, hybridisiert mit der ES 13-Sonde.
Spuren 5 und 6: Mapping der Nukleinsäure von 10 000 und 100 000 Zellen, transfiziert mit ES 53, das mit der ES 53-Sonde hybridisiert worden war.

### Beispiel 6

Schwächung der Wirkung des v-erb B Onkogens durch tDNA Ribozym-Gene, die mit Hilfe von Polylysin-Transferrin - Konjugaten in v-erb B transformierte Erythroblasten eingebracht wurden.

Anhand dieses Beispiels konnte gezeigt werden, daß tDNA-Ribozyme, die gegen das erb B Onkogen gerichtet sind, mit Hilfe von Polylysin-Transferrin-Konjugaten in erb B-transformierte Hühner-Erythroblasten eingebracht werden und die transformierende Wirkung des Onkogens schwächen können.

### Vorversuch 1

### Herstellung von Transferrin - Polylysin - Konjugaten

Die Kopplung erfolgte analog literaturbekannter Methoden (vgl. G. Jung, W. Köhnlein und G. Lüders, Biochem. Biophys.Res. Commun. 101 (1981), 599 ) durch Einführung von Disulfidbrücken nach Modifizierung mit Succinimidylpyridyldithiopropionat.

### Pyridyldithiopropionat - modifiziertes Transferrin 1:

6 ml einer über Sephadex G-25 gelfiltrierten Lösung von 120 mg ( 1.5 µmol) Transferrin ( aus Hühnereiweiß, Sigma, Conalbumin Type I, eisenfrei) in 0.1 M Natriumphosphat-Puffer ( pH 7.8) wurden unter gutem Schütteln mit 200 µl einer 15 mM ethanolischen Lösung von Succinimidylpyridyldithiopropionat (SPDP, Pharmacia) versetzt und 1 h bei Raumtemperatur und gelegentlichem Schütteln reagieren gelassen. Über eine Gelsäule (Sephadex G-25, 14 x 180 mm, 0.1 M Natriumphosphat-Puffer pH 7.8 ) wurden niedermolekulare Reaktionsprodukte und Reagensreste abgetrennt und dabei 7 ml der Produktfraktion erhalten; der Gehalt von an Transferrin gebundenen Pyridyldithiopropionatresten wurde anhand eines Aliquots nach Reduktion mit Dithiothreitol durch photometrische Bestimmung der Menge an freigesetztem Pyridin-2-thion ermittelt und betrug ca. 2.6 µmol.

### Mercaptopropionat - modifiziertes Polylysin 2:

Eine Lösung von 18 mg (ca. 1.0 µmol) Poly(L)Lysin - Hydrobromid (Sigma, Fluoresceinisothiocyanat ( = FITC ) - markiert, Molekulargewicht ca. 18000 / entspricht einem durchschnittlichem Polymerisationsgrad ca.90 ) in 3 ml 0.1 M Natriumphosphat (pH 7.8) wurde über Sephadex G-25 filtriert. Die Polylysin-Lösung wurde mit Wasser auf 7 ml verdünnt, unter gutem Schütteln mit 270 µl einer 15 mM ethanolischen Lösung von SPDP versetzt, und 1 h in der Dunkelheit bei Raumtemperatur und unter gelegentlichem Schütteln reagieren gelassen. Nach Zugabe von 0.5 ml 1 M Natriumacetat-Puffer (pH 5.0) wurde zur Abtrennung von niedermolekularen Substanzen über Sephadex G-25 filtriert (Eluens : 20 mM Natriumacetat-Puffer pH 5.0). Die Produktfraktion (Ninhydrinfärbung, Fluoreszenz) wurde i.Vak. eingeengt, mit Puffer auf pH ca. 7 gebracht, eine Lösung von 23 mg (150 µmol) Dithiothreitol in 200 µl Wasser zugegeben und 1 h bei Raumtemperatur, unter Argon in der Dunkelheit stehen gelassen. Durch weitere Gelfiltration (Sephadex G-25, 14 x 130 mm Säule, 10 mM Natriumacetat-Puffer pH 5.0) wurde von überschüssigem Reduktionsmittel abgetrennt und man erhielt 3.5 ml Produktlösung an Fluoreszenz-markiertem Polylysin mit einem Gehalt von 3.8 mol Mercaptogruppen (photometrische Bestimmung mittels Ellman's Reagens, 5,5'-Dithiobis(2-nitrobenzoesäure)).

### Transferrin - Polylysin - Konjugate 3:

Die wie oben beschrieben erhaltene Lösung von modifiziertem Transferrin 1 ( 7 ml in 0.1 M Natriumphosphat-Puffer pH 7.8 , ca. 1.5 µmol Transferrin mit ca 2.6 µmol Pyridyldithiopropionat-Resten) wurde mit Argon gespült; es wurden 2.0 ml der oben beschriebenen Lösung von Mercapto-modifiziertem Polylysin 2 (in 10 mM Natriumacetat-Puffer pH 5.0, das entspricht ca 0.6 µmol Polylysin mit ca 2.2 µmol Mercaptogruppen) zugegeben, mit Argon gespült, geschüttelt und 18 h bei Raumtemperatur in der Dunkelheit und unter Argon reagieren gelassen. Das Reactionsgemisch wurde mit Wasser auf 14 ml verdünnt und durch Ionenaustauschchromatographie aufgetrennt (Pharmacia Mono S Säule HR 10/10, Gradienteneluation, Puffer A : 50 mM HEPES pH 7.9 , Puffer B : A + 3 M Natriumchlorid, 0.5 ml/min). Nichtkonjugiertes Transferrin wurde am Anfang eluiert, Produktfraktionen bei ca. 0.66 - 1.5 M Natriumchlorid. Die konjugierten Produkte (Ninhydrinfärbung, in UV bei 280 nm Proteinabsorbtion, und Fluoreszenz) wurden in 6 Fraktionen mit einem Gehalt von je ca. 10 mg Transferrin gesammelt. Die Fraktionen wurden zunächst gegen eine 100 mM Eisen(III)citrat-Lösung (mit Natriumhydrogencarbonat auf pH 7.8 gebracht) dialysiert und danach noch zweimal gegen 1 mM HEPES-Puffer (pH 7.5).
Natriumdodecylsulfat-Gelelektrophorese (10% SDS, 8% Polyacrylamid) zeigte bei Vorbehandlung mit 2-Mercaptoethanol in allen 6 Fraktionen einen ungefähr gleichen Gehalt an Transferrin , während in nicht reduzierten Proben keine Banden für freies Transferrin, sondern nur weniger weit wandernde Konjugate sichtbar waren.

### Vorversuch 2

### Transport von Transferrin-Polylysin-Konjugaten in lebende Zellen

Um nachzuweisen, daß die im Vorversuch 1 beschriebenen Transferrin-Polylysin-Konjugate effizient in lebende Erythroblasten aufgenommen werden, wurden diese Konjugate mit FITC markiert. Es ist bekannt (Schmidt et al., 1986), daß FITC- markiertes Transferrin nach einigen Stunden Inkubation mit Erythroblasten, denen vorher Transferrin entzogen worden war, in Vesikeln innerhalb der Zelle nachweisbar war (Untersuchung im Fluoreszenzmikroskop).

Im vorliegenden Beispiel wurden Erythroblasten, (durch ein EGF-Rezeptor-Retrovirus transformiert, Kahazaie et al., 1988) 18 Stunden in transferrinfreiem Differenzierungsmedium (Zusammensetzung in Zenke et al., 1988) bei 37°C (Zellkonzentration 1,5x10⁶/ml) inkubiert. Nach Zugabe der verschiedenen Transferrin-Polylysin-Konjugate (oder, als Kontrolle, der entsprechenden Menge sterilen aq bidest) wurden die Zellen bei 37°C in Gegenwart von 10 ng/ml EGF (um den transformierten Zustand aufrechzuerhalten) inkubiert. Nach 24 und 48 Stunden wurden ca. 5x10⁵ Zellen entnommen,1 mal in phosphatgepufferter physiologischer Kochsalzlösung (PBS; pH 7.2) gewaschen, mit dem 50fachen Volumen einer Mischung von 3.7 % Formaldehyd und 0.02% Glutaraldehyd in PBS fixiert (10 min, 40°C), 1 mal in PBS gewaschen, in Elvanol eingebettet und im Fluoreszenzmikroskop (Zeiss Axiophot,Schmalband-FITC und TRITC-Anregung) untersucht. Gleichzeitig wurde in anderen Aliquots der verschiedenen Ansätze die Wachstumsrate der Zellen bestimmt. Hierzu wurden 100 µl Zellsuspension entnommen und der Einbau von ³H-Thymidin (8 µCi/ml,2 Stunden) bestimmt, wie in Leutz et al., 1984 beschrieben. Aus Fig. 10 geht hervor, daß die mit Transferrin-Polylysin inkubierten Erythroblasten nach 24 Stunden 2 bis 10 stark fluoreszierende Vesikel aufweisen, die in den Kontrollen nicht nachweisbar sind. Tabelle A zeigte, daß mit Ausnahme der Fraktion 6 alle Konjugate von praktisch allen Zellen aufgenommen worden sind.

Die Tatsache, daß die Zellen in allen Proben gleich schnell wachsen (wie durch Einbau von tritiiertem Thymidin (³H TdR) gemessen; Tabelle A), beweist, daß die Zellen durch die Polylysin-Konstrukte nicht geschädigt werden und somit eine unspezifische Aufnahme (z.B. über durchlässig gewordene Zellmembranen) auszuschließen ist.

### Vorversuch 3

### Polylysin-Transferrinkonstrukte können bei der in vitro induzierten Reifung von Hühner-Erythroblasten zu Erythrocyten den nativen Transferrin-Eisenkomplex funktionell ersetzen

Ziel dieses Versuches war es zu zeigen, daß die hier verwendeten Transferrin-Polylysin-Konjugate von der Zelle wie natives Transferrin verwendet werden, d.h. den normalen Transferrinzyklus mit ähnlicher Effizienz durchlaufen. Als Testsystem hierfür sind Erythroblasten, die durch "Abschalten" des transformierenden Onkogens zur Ausreifung in normale Erythrocyten induziert werden können, besonders geeignet (Beug et al., 1982). Aus der Literatur geht hervor, daß solche Zellen zur normalen Reifung hohe Konzentrationen an Transferrin-Eisen-Komplex benötigen (100 - 200 µg/ml, 3 fach geringere Konzentrationen verhindern das Ausreifen der Zellen und führen nach mehreren Tagen zum Tod der Zellen (Kowenz et al., 1986). Auch konnte gezeigt werden (Schmidt et al., 1986), daß "recycling", also Wiederverwendung von Transferrinrezeptoren und damit ein mit optimaler Geschwindigkeit ablaufender Transferrinzyklus für eine normale in vitro Differenzierung unerläßlich ist.

Erythroblasten (durch das EGF-Rezeptor-Retrovirus transformiert) wurden durch Entzug von EGF und Zugabe einer optimalen Menge an partiell gereinigtem Hühner-Erythropoietins (Kowenz et al., 1986, frei von Transferrin) zur Differenzierung induziert. Die Inkubation erfolgte bei einer Zellkonzentration von 1x10⁶/ml in transferrinfreiem Differenzierungsmedium bei 42°C und 5% CO₂. Bei Inkubationsbeginn wurde entweder nativer Transferrin-Eisen-Komplex (Sigma, 100 µg/ml) oder die mit Eisen gesättigten Transferrin-Polylysin-Konjugate (Konzentration ebenfalls 100 ug/ml) zugegeben. Wachstum und Reifungszustand der Zellen wurden auf folgende Art nach 24 und 48 h analysiert:
1. Bestimmung der Zellzahl (im Coulter Counter, Modell ZM, Beug et al,1984)
2. Aufnahme von Zell-Größenverteilungen (im Coulter-Channelyzer Mod. 256) und
3. Photometrische Messung des Hämoglobingehalts der Zellen (Kowenz et al., 1986)
Außerdem wurden Aliquots der Ansätze nach 72 Stunden in einer Cytozentrifuge (Shandon) auf Objektträger zentrifugiert und einem histochemischen Nachweis für Hämoglobin unterzogen (Färbung mit neutralem Benzidin plus Diff-Quik Schnellfärbung für Blutzellen, Beug et al., 1982).

Die Ergebnisse in Tabelle B zeigen deutlich, daß Zellen, die in Gegenwart der Polylysin-Transferrin Konjugate Fraktion 1 bis 5 zur Differenzierung induziert wurden, genauso effizient und mit gleicher Geschwindigkeit ausreifen wie solche, die mit nativem Transferrin-Eisen inkubiert wurden. Die Zellen in den transferrinfreien Kontrollen dagegen zeigten ein stark verlangsamtes Zellwachstum und akkumulierten nur geringe Mengen an Hämoglobin. Die Untersuchung des Zellphänotyps an gefärbten Cytospin Praeparaten ergab, daß die mit Polylysin-Transferrin-Konjugaten inkubierten Zellen genauso zu späten Retikulocyten (late reticulocytes, Beug et al.,1982) herangereift waren wie die mit nativem Transferrin behandelten, während die ohne Transferrin inkubierten Zellen eine Mischung aus desintegrierten und unreifen, erythroblastenähnlichen Zellen darstellten (Schmidt et al., 1986). Nur die mit Transferrin-Polylysin-Fraktion 6 behandelten Zellen wiesen einen geringeren Hämoglobingehalt und einen größeren Prozentsatz an unreifen Zellen auf (Tabelle B). Dies zeigt, daß die mit besonders viel Polylysin konjugierte Fraktion 6 weniger gut im Transferrinzyklus funktioniert. Gleichzeitig weist dieses Ergebnis auf die Empfindlichkeit der Testmethode hin.

### Vorversuch 4

### Polylysin-Transferrin-Konjugate ermöglichen die Aufnahme von DNA in Hühnererythroblasten

In vorliegendem Versuch sollte untersucht werden, ob DNA in einer Größe, die der von tDNA-Ribozymen (siehe Beispiel 1) entspricht, von Transferrin-Polylysin-Konjugaten effizient in das Zellinnere transportiert werden kann. Im vorliegenden Beispiel wurde tDNA mit einem Insert der Sequenz

CGTTAACAAGCTAACGTTGAGGGGCATGATATCGGGCC
CCGGGCAATTGTTCGATTGCAACTCCCCGTACTATAGC,

Molekulargewicht ca. 300.000, mit gamma ³²P ATP endmarkiert (Maniatis), verwendet. Ungefähr 0.3 µg dieser DNA, gelöst in 20 µl TE Puffer, wurden entweder mit 10 µg nativem Transferrin, mit 10 µg Transferrin-Polylysin Konjugat Fraktion 3, jeweils gelöst in 50 µl aq bidest plus 400 µg/ml Rinderserumalbumin (Beug et al., 1982) oder mit 50 µl dieses Lösungsmittels ohne Transferrin vermischt. Die DNA-Proteinmischungen wurden zu je 2 ml transferrinfreiem Differenzierungsmedium zugegeben, 4x10⁶ Hühnererythroblasten, (die mit einem EGF-Rezeptor-Retrovirus transformiert waren und 18 h. in transferrinfreiem Medium in Gegenwart von EGF vorinkubiert wurden, (Kahazaie et al., 1988) zugegeben und die Ansätze 8 Stunden bei 37°C und 5 % CO₂ inkubiert. Danach wurden die Zellen abzentrifugiert, der Überstand abgenommen und die Zellen 3 mal in transferrinfreiem Medium gewaschen. Zellsediment und Kulturmedium wurden in 1% SDS,1 mg/ml Proteinase K, 300 mM NaCl, 20 mM Tris pH 8.0, 10 mM EDTA (PK/SDS-Puffer) aufgenommen, 30 min bei 37°C inkubiert, mit Phenol/Chloroform extrahiert, und die DNA durch Ethanolfällung isoliert. Isolierte DNA mit einer Radioaktivität von insgesamt 2000 cpm wurde auf einem nicht-denaturierenden 3.5 % Acrylamidgel aufgetrennt (TBE, Maniatis) und die DNA durch Autoradiographie nachgewiesen. Die Fig. zeigt Fluoreszenzaufnahmen von Hühnererythroblasten, die 24 h ohne (A) oder mit FITC-markierten Transferrin-Polylysin-Konjugaten (B,C) inkubiert wurden. Bei Anregung mit blauem Licht (B, zum Nachweis von FITC) sind deutlich mehrere fluoreszierende Vesikel in jeder Zelle zu erkennen. Die Spezifität dieser Fluoreszenz wird dadurch gezeigt, daß die Vesikelfluoreszenz bei Anregung mit grünem Licht (bei welcher eine ähnliche unspezifische Fluoreszenz der Zellen wie in A zu sehen ist) nicht auftritt (C).

Diese Fig. zeigt, daß in der mit Transferrin-Polylysin behandelten Zellprobe etwa 5-10 mal mehr DNA von den Zellen aufgenommen worden ist als in den Kontrollproben mit nativem Transferrin oder ohne Transferrin.

Zwei tRNA-Ribozymgene, gerichtet gegen die Translationsinitions-Region von erbB wurden konstruiert (vgl. Fig.2 und 3, Beispiel 1). Ca. 100 µg jedes das Gen enthaltenden Plasmids wurden EcoRI verdaut, um das tRNA-Ribozymgen auf einem 225 bp-Fragment freizusetzen. Die Verdauprodukte wurden mittels Klenow-Fragment endmarkiert und mittels Gelelektrophorese durch ein 2% Agarose/TBE-Gel gereinigt. Das Vektorfragment und die tRNARibozymgen-Fragmente wurden durch Ethidiumbromid-Färbung lokalisiert, ausgeschnitten und durch Elektroelution, Phenol/Chloroform- und Chloroformextraktion und Ethanolfällung gewonnen. Die gereinigten, radioaktiv markierten DNA-Fragmente wurden daraufhin unter Benutzung des Transferrin-Polylysin-Transportsystems dazu verwendet, die Aufnahme und die Hemmung der erbB-RNA zu bestimmen. Als Kontroll-DNA wurde der Vektor pSPT 18 verwendet.

Als Testzellsystem wurde eine Hühnererythroblastenzellinie gewählt, die durch eine temperatursensitve Mutante (ts 34, Graf et al., 1978) des Vogel- Erythroblastosevirus AEV transformiert ist (Beug et al., 1982 b).(Das in diesen Zellen ebenfalls exprimierte erb A Onkogen kann durch einen spezifischen Proteinkinase-Hemmer (H 7) inhibiert werden. Es wurde festgestellt, daß das v-erbA-Onkogen in vivo und in vitro (d.h. als bakteriell exprimiertes Protein) an zwei Stellen, nämlich Ser28 und Ser29, durch Proteinkinase C bzw. durch cAMP-abhängige Proteinkinase phosphoryliert wird. Mutation dieser Serine zu Alaninen verhindert die Phosphorylierung und zerstört die v-erbA-Onkogenaktivität. H7 ist ein spezifischer Inhibitor dieser beiden Kinasen und ist in der Lage, in v-erbA-v-erbB haltigen Erythroblasten selektiv die durch v-erbA verursachten Veränderungen (z.B. Blockierung der Differenzierung) aufzuheben).

Es ist bekannt, daß Erythroblasten, deren erbB Onkogen inaktiviert wird - z.B. durch Erhöhung der Temperatur im Falle einer temperaturempfindlichen erbB Mutante - dazu induziert werden, zu Erythrocyten auszureifen. Eines der ersten Anzeichen für diesen Vorgang ist eine Induktion der Hämoglobinsynthese, die durch einen empfindlichen Nachweis (saure Benzidinfärbung, Orkin et al., 1975, Graf et al., 1978) auf dem Niveau der Einzelzelle nachgewiesen werden kann. Als phänotypische Wirkung eines gegen erbB gerichteten Ribozyms in diesem Testsystem wäre somit eine spezifische Erhöhung der Zahl Benzidin-positiver Zellen zu erwarten.

Die diesem Beispiel zugrundeliegende Versuchsreihe wurde folgendermaßen durchgeführt: Die verschiedenen DNA-Präparationen (siehe oben und Tabelle C), gelöst in 30 µl TE-Puffer, wurden mit jeweils 10 µg nativem Transferrin-Eisenkomplex oder Transferrin-Polylysin - Konjugat (gelöst in 50 µl aq. bidest.) gemischt und 30 min bei 37°C inkubiert.

Im Falle der Vektor-DNA (10 µg) wurde entsprechend mehr (100 µg) an Transferrin-Präparationen verwendet. Die DNA-Transferrin-DNA-Mischungen -Mischungen wurden zu je 1 ml transferrinfreiem Differenzierungsmedium (Zenke et al., 1988) zugegeben. Die Testzellen (pro Ansatz 3x10⁶) wurden vor dem Versuch 60 min in transferrinfreiem Differenzierungsmedium bei 42°C inkubiert, (hierdurch wird die Transferrinaufnahme verstärkt) und zu den DNA-Transferrin-haltigen Ansätzen hinzugegeben. Nach 6 h, 18 h und 68 Stunden (Behandlung der Zellen siehe unten) wurden Proben entnommen, wie beschrieben, in Überstand und Zellsediment aufgetrennt, in PK/SDS Puffer aufgenommen und die DNA analysiert (Fig. 9).

Nach Inkubationsende (6 h) wurden die Zellen abzentrifugiert und in transferrinhaltigem Differenzierungsmedium mit Erythropoietin und Insulin (Kowenz et al., 1986, Zenke et al., 1988), 2 ml pro Ansatz und bei 37°C d.h. in Anwesenheit eines aktiven v-erb B-Proteins) weitere 72 h inkubiert.

Folgende Ergebnisse wurden erhalten:
1. Ähnlich wie in Vorversuch 4 konnte eine verstärkte Aufnahme von DNA in der Größe der erbschnitt DNAs in den mit Transferrin-Polylysin behandelten Zellproben (etwa 5 fach) beobachtet werden.
2. Durch Transfektion von Hühnerfibroblasten mit erbschnitt DNA konnte gezeigt werden, daß die erbschnitt-Ribozym-tDNA in Hühnerzellen exprimiert wird (siehe Beispiel 5).
3. Tabelle C zeigt, dass in allen Fällen, in denen erbschnitt-Ribozym-tDNA mit Hilfe von Polylysin-Transferrin-Konstrukten in erb B transformierte Erythroblasten eingebracht wurde, der Prozentsatz an Benzidin-positiven Zellen signifikant (auf ca. das Doppelte) erhöht war (als Referenz dienten die Proben, die mit Vektor-DNA behandelt wurden und in denen die Verwendung von Polylysin-Transferrin-Konjugaten erwartungsgemäß nicht zu einer Erhöhung der Zahl Benzidin-positiver Zellen führte).

### Beispiel 7

### Verlängerung der Anticodon-Stammregion erhöht die Ausbeute an ribtRNA

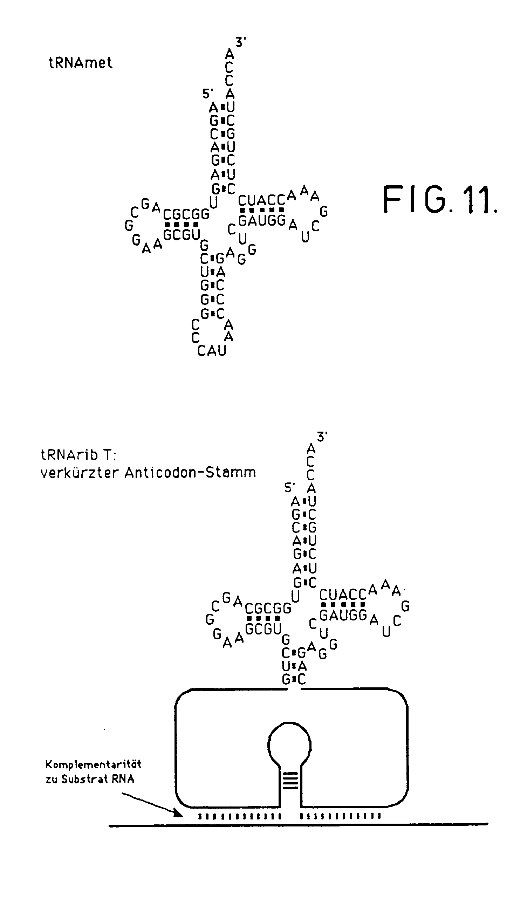

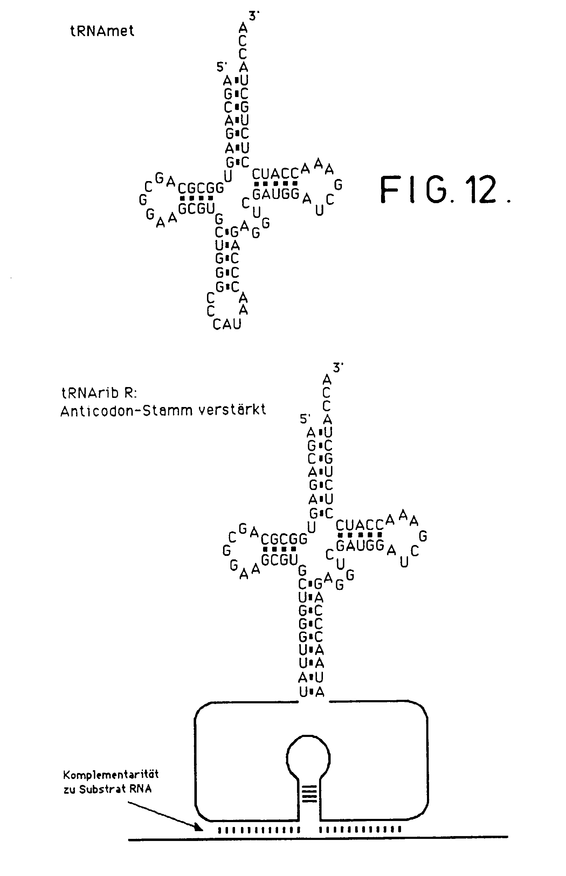

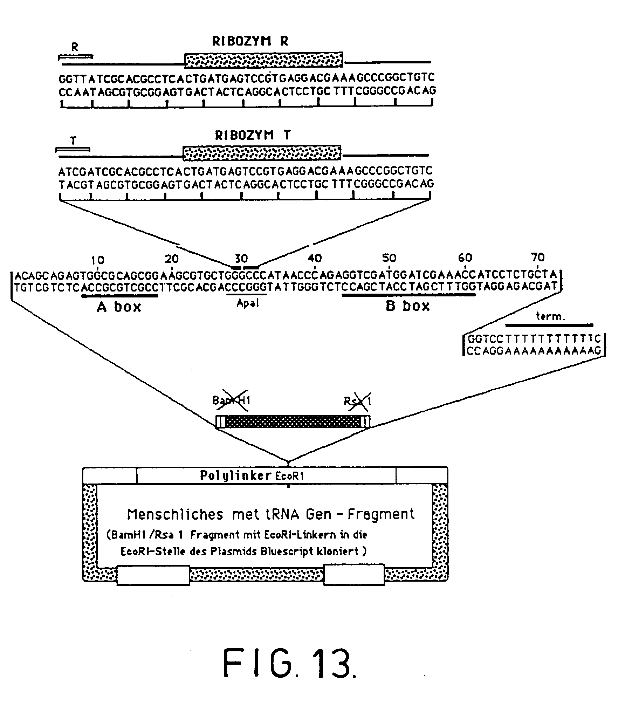

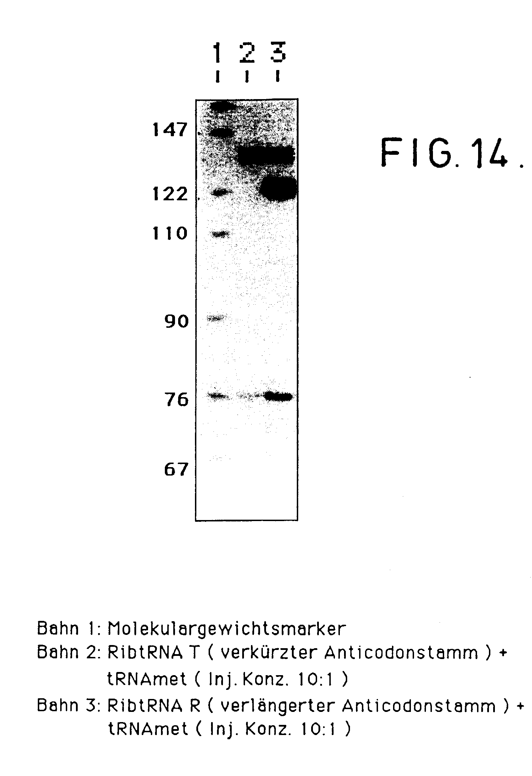

Das humane met tRNA-Gen (kloniert als BamHI/RsaI Fragment, ergänzt durch EcoRI Linker, in die EcoRI Schnittstelle des Bluescript-Vektors) wurde an seiner einzigen ApaI-Schnittstelle in der Anticodonstammregion gespalten. Die einzelsträngigen Überhänge wurden durch T4-DNA-Polymerasebehandlung entfernt und Oligonukleotide, enthaltend die Ribozymsequenzen, inseriert. Die in den vorangegangenen Beispielen verwendete Ribozyminsertion resultierte in einem ribtRNA-Molekül, enthaltend einen 3-Basen-Stamm (Fig. 11 zeigt die Wildtyp-tRNAmet und die tRNArib mit dem verkürzten Anticodon-Stamm). Bei der zweiten Konstruktion wurde ein Oligonukleotid, das sich vom ersten durch die zur Wildtypsequenz komplementäre Sequenz GGTTAT am 5′Ende unterschied, verwendet. Dabei wurde der Wildtypstamm wiederhergestellt und durch 4 zusätzliche Basenpaare verlängert (Fig. 12 zeigt die Konstruktion der tRNArib mit dem verstärkten Anticodon-Stamm. Die Sequenz der Ribozyme ist in Fig. 13 dargestellt; am linken (5′) Ende sind die Unterschiede in der Nukleotidsequenz zwischen der für den verkürzten Stamm und den verstärkten Stamm kodierenden Region gekennzeichnet. In Fig.13 ist weiters die Sequenz des met tRNA-Gens wiedergegeben). Die beiden Ligationsprodukte wurden nach der in Maniatis beschriebenen Standardmethode in E.coli HB 101 transformiert, geeignete Klone wurden ausgewählt, die Plasmid DNA isoliert und zur Bestimmung der richtigen Struktur sequenziert. Die beiden resultierenden ribtRNA-Gene wurden durch Mikroinjektion der klonierten DNA in Xenopus Oozyten, durchgeführt wie in Beispiel 2 bzw. 3 beschrieben, in Gegenwart von ³²P-GTP auf Transkriptionsaktivität und Akkumulierung der ribtRNA-Moleküle untersucht. Das Wildtyp-tRNA-Gen wurde bei 10fach niedrigerer Konzentration coinjiziert. Die injizierten Oozyten wurden 7 h lang bei 20°C inkubiert, die resultierende RNA wurde geerntet (vgl Beispiel 2) und elektrophoretisch aufgetrennt (10 % Acrylamid/8,3 M Harnstoff/TBE-Gel) und die RNA-Moleküle durch Autoradiographie (2 Tage exponiert bei -70°C) sichtbar gemacht (Fig.14). Das verkürzte ribtRNA-Ge liefert ca. 1/10 der RNA-Menge, die vom Wildtyp-Gen transkribiert wird, im Vergleich dazu liefert das Gen, das für das ribtRNA Molekül mit dem verlängerten Stamm kodiert, die 6fache Menge an RNA.

### Beispiel 8

### Expression von Ribozymgenen als Bestandteil von Introns

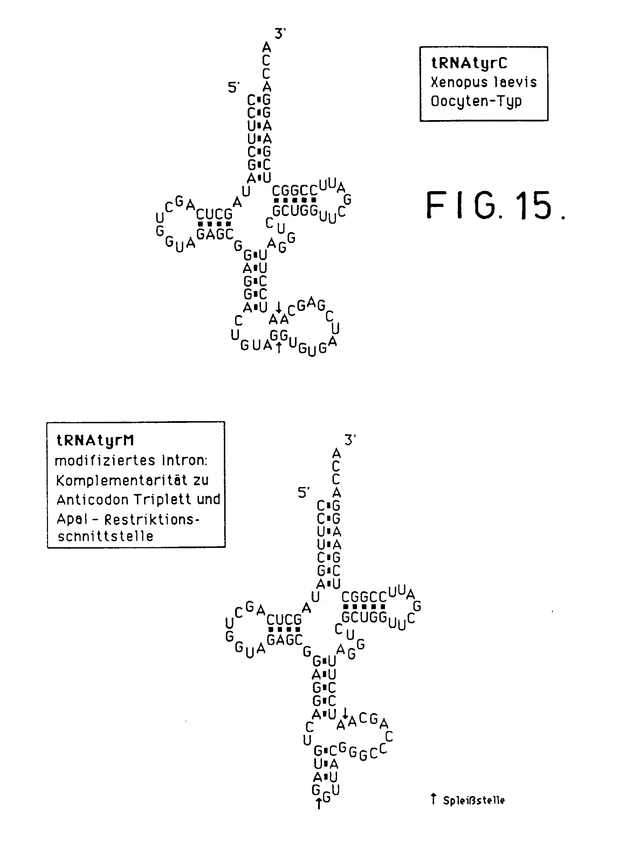

Als Ausgangsgen wurde ein Xenopus laevis tRNA^{tyr}C-Gen (Oocyten-Typ) gewählt, welches ein 13 Nukleotide umfassendes Intron enthält. Die natürliche Intron-Sequenz wurde wie folgt modifiziert: Erstens wurde eine geeignete Restriktionsschnittstelle eingefügt (Apal; GGGCCC), um eine nachfolgende Klonierung von Oligonukleotiden zu ermöglichen, zweitens komplementäre Nukleotide zum Anticodon-Triplett, um durch Verlängerung der Intronsequenz über ein zusätzlich stabilisierendes Strukturmerkmal zu verfügen. Die Größe des Introns im modifizierten Gen ist von 13 Nukleotide auf 15 Nukleotide erhöht (Fig. 15).

Die Modifizierung der Intronsequenz wurde mit Hilfe der Polymerase-Kettenreaktion ("Polymerase chain reaction" PCR; Ho et.al.,1989) durchgeführt. Dafür wurden vier Primer synthetisiert, von denen zwei die geänderte Intronsequenz (zueinander komplementär) enthielten und zwei gegen das 5′- bzw. das 3′-Ende des Gens gerichtet waren, um eine EcoRI bzw. Sall Restriktionsschnittstelle einzuführen. Das Wildtyp-Gen lag als Hhal-Fragment (258 bp), kloniert in pBR327, vor (Stutz et al.1989). Das resultierende PCR-Produkt wurde über ein Agarosegel gereinigt, mit den genannten Restriktionsenzymen geschnitten und mit dem Vektor pAALM (= pSP64 + T7 Promotor; Vieira und Messing, 1982) ligiert. Das Konstrukt wurde in E.coli HB 101 transformiert und Klone, enthaltend das gewünschte Insert, durch Sequenzanalyse identifiziert.

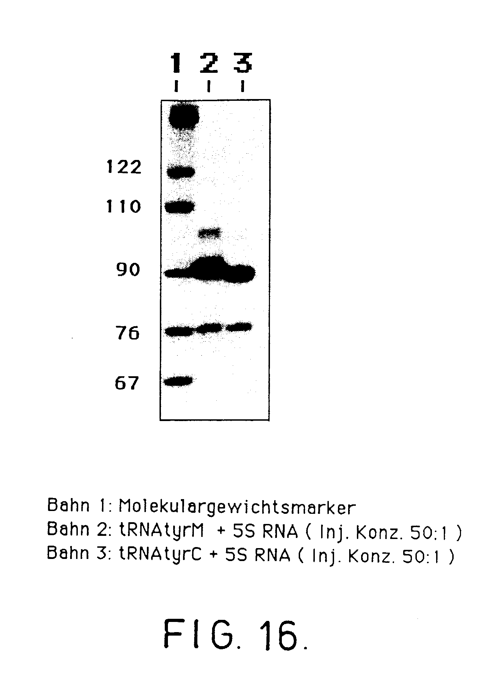

Die Aktivität des modifizierten Gens (tRNA^{tyr}M) wurde mit der des Wildtyp-Gens durch Mikroinjektion in Xenopus Oocyten verglichen, wobei als interner Standard ein 5S-RNA-Gen (50 fach niedrigere Konzentration),das auf dem Plasmid pUC-10-5S vorlag (Carroll und Brown, 1976) in Gegenwart von ³²P-GTP coinjiziert wurde. Die injizierten Oocyten wurden 20 Stunden bei 20°C inkubiert, die RNA auf einem 8% Acrylamid/8,3 M Harnstoff/TBE-Gel aufgetrennt und autoradiografiert (Fig. 16). Sichtbar werden neben der 5S-RNA bei 120 nt das primäre tRNA^{tyr}-Transkript bei 100 (102) nt, die 5′- und 3′- prozessierte Precursorform bei 90 (92) nt, sowie die fertig prozessierte Tyrosin tRNA bei 76 nt. Das Spleißen der 90 nt Precursorform erscheint als der limitierende Faktor, sodaß der Großteil des gebildeten Transkripts (ca. 80 %) in dieser Form vorliegt. Wie erwartet, wurde die biologische Aktivität durch die Modifikation gegenüber dem Wildtyp-Gen nicht vermindert.

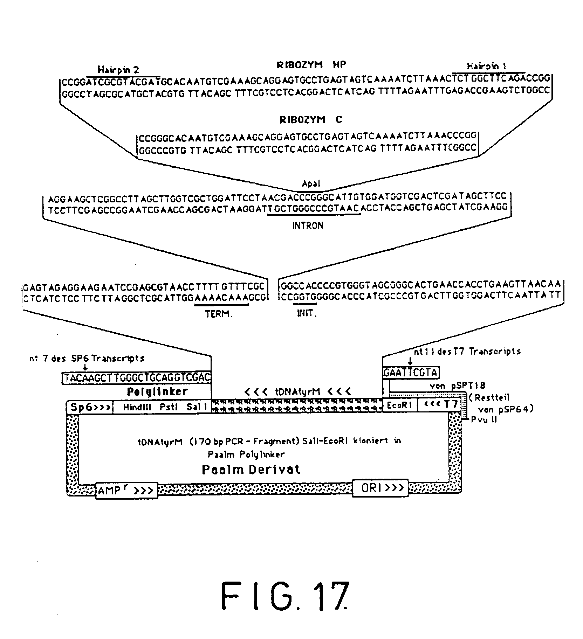

In einem weiteren Experiment wurde die Leistungsfähigkeit des Systems getestet. Es wurden zwei Ribozymsequenzen enthaltende Oligodeoxyribonukleotide synthetisiert, die bereits Apal-Enden enthalten und somit direkt in die Intronsequenz des modifizierten tRNA^{tyr}-Gens kloniert werden konnten. Bei einem Oligonukleotid wurden an beiden Enden 12 nt eingefügt, um in Ribintrons stabile "hairpins" zu bilden und damit Exonukleaseabbau entgegenzuwirken. Die Gesamtgröße des daraus resultierenden Introns betrug 89 nt (Ribozym HP) gegenüber 65 nt im Falle der ungeschützten Ribozymsequenz (Ribozym C). Die Sequenzen der Ribozyme sowie das Klonierungsschema sind in Fig.17 dargestellt.

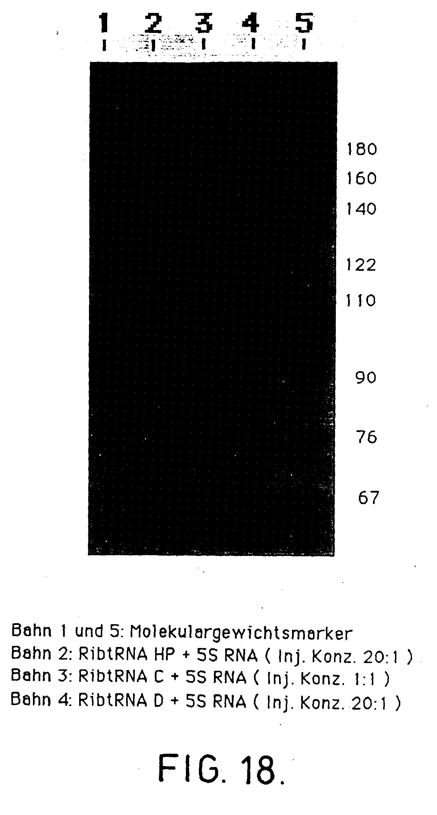

Für die analog zu den oben beschriebenen Mikroinjektionen in Xenopus Oocyten wurde neben den beiden beschriebenen Konstrukten ein drittes verwendet, welches das Ribozym HP in dimerer Form enthält und so die Introngröße auf 163 nt erhöht (Ribozym D). Die Konzentration des coinjizierten 5S-Standards betrug 1 : 20 bei den Konstrukten HP und D, 1 : 1 bei Konstrukt C (Fig.18). Das Experiment zeigt, daß die Konstrukte HP und C trotz wesentlich vergrößerter Introns sehr aktiv transkribiert und mit gleicher Effizienz prozessiert werden wie das Wildtyp tRNA^{tyr}-Gen. Im Falle des Konstrukts D ist nur eine minimale Menge Transkript nachweisbar, weil offensichtlich die für die Bildung eines PoI III - Transkriptionskomplexes notwendige Sekundärstruktur durch die lange Intronsequenz zerstört wurde.

Es konnte gezeigt werden, daß die Expression von Ribozymen als Introns von tRNAs zu keiner wesentlichen Beeinträchtigung der tRNA-Sekundärstruktur führt, wodurch das entstehende Transkript in hoher Konzentration akkumuliert und korrekt prozessiert werden kann.

### Literatur:

Altmann et al. (1988), Structure and Function of Major and Minor Small Nuclear Ribonucleoprotein Particles, Herausgeber Birnstiel, Springer Verlag, Berlin, 183-195
Beug et al. (1982a), J.Cell Physiol. suppl.1, 195-207
Beug et al. (1982b), Cell 28, 907-919
Beug et al. (1984), Cell 36, 963-972
Caroll and Brown, (1976), Cell 7, 477-486
Ciliberto et al. (1982), Proc.Natl.Acad.Sci.79, 1921-1925
Clarkson, Eukaryotic Genes, Their Structure, Activity and Regulation, Herausgeber: McLean et al., Butterworth (1983), 239-261
Cotten et al. (1988), EMBO J., 7, 801-808
Felgner et al. (1987), Proc.Natl.Acad.Sci. 84, 7413-7417
Folk et al. (1983), Cell 33, 585-593
Fromm et al. (1987), Methods in Enzymol. 153, 351-366
Geiduschek et al. (1988), Ann.Rev.Biochem. 57, 873-914
Graf et al. (1978), Nature 275, 496-501
Graham et al. (1973), Virology 52, 456-467
Hampel u.Tritz (1989), Biochemistry 28, 4929-4933
Haseloff et al. (1988), Nature 334, 585-591
Ho et al. (1989), Gene 77, 51-59
Hofstetter et al. (1981), Cell 24, 573-585
Jennings et al. (1987), EMBO J. Vol.6, 10, 3043-3047
Jung et al. (1981), Biochem.Res.Commun.101, 599
Kahazaie et al. (1988), EMBO J. 10, 3061-3071
Kowenz et al. (1986), Mod.Trends in Human Leukemia VII, Springer Verlag, 199-209
Kressmann et al. (1978), Proc.Natl.Acad.sci. 75, 1176-1180
Kressmann et al. (1980), Series 31, 383-407
Kuo et al. (1988), J.Virol. 62, 4439-4444
Langridge et al. (1987), Methods Enzymol. 153, 336-351
Leutz et al. (1984), EMBO J. 3, 3191-3197
Maniatis et al. (1982), Molecular Cloning, Cold Spring Harbor
Melton et al. (1984), Nucleic Acids Res. 12, 7035-7056
Mowry et al. (1987), Science 238, 1682-1687
Orkin et al. (1975), Proc.Natl.Acad.Sci. 72, 98-102
Pepperkok et al. (1988), Proc.Natl.Acad.Sci.85, 6748-6752
Schmidt et al. (1986), Cell 46, 41-51
Sharmeen et al. (1988), J.Virol.62, 2674-2679
Soldati et al. (1988), Mol.Cell.Biol.8, 1518-1524
Stewart et al. (1987), EMBO J.6, 383-388
Stutz et al. (1989), Genes & Development Vol.3, 8, 1190-1198
Tellford et al. (1979), Proc.Acad.Sci. 76, 2590-2594
Uhlenbeck et al. (1987), Nature 328, 596-600
Vennstrom et al. (1980), J.Virol. 36, 575-585
Viera und Messing, (1982), Gene 19, 259-268
Wu et al. (1989), Proc.Natl.Acad.Sci. 86, 1831-1835
Zasloff et al. (1982), Nature 300, 81-84
Zasloff et al. (1983), Proc.Acad.Sci. 80, 6426-6440
Zenke et al. (1988), Cell 52, 107-119

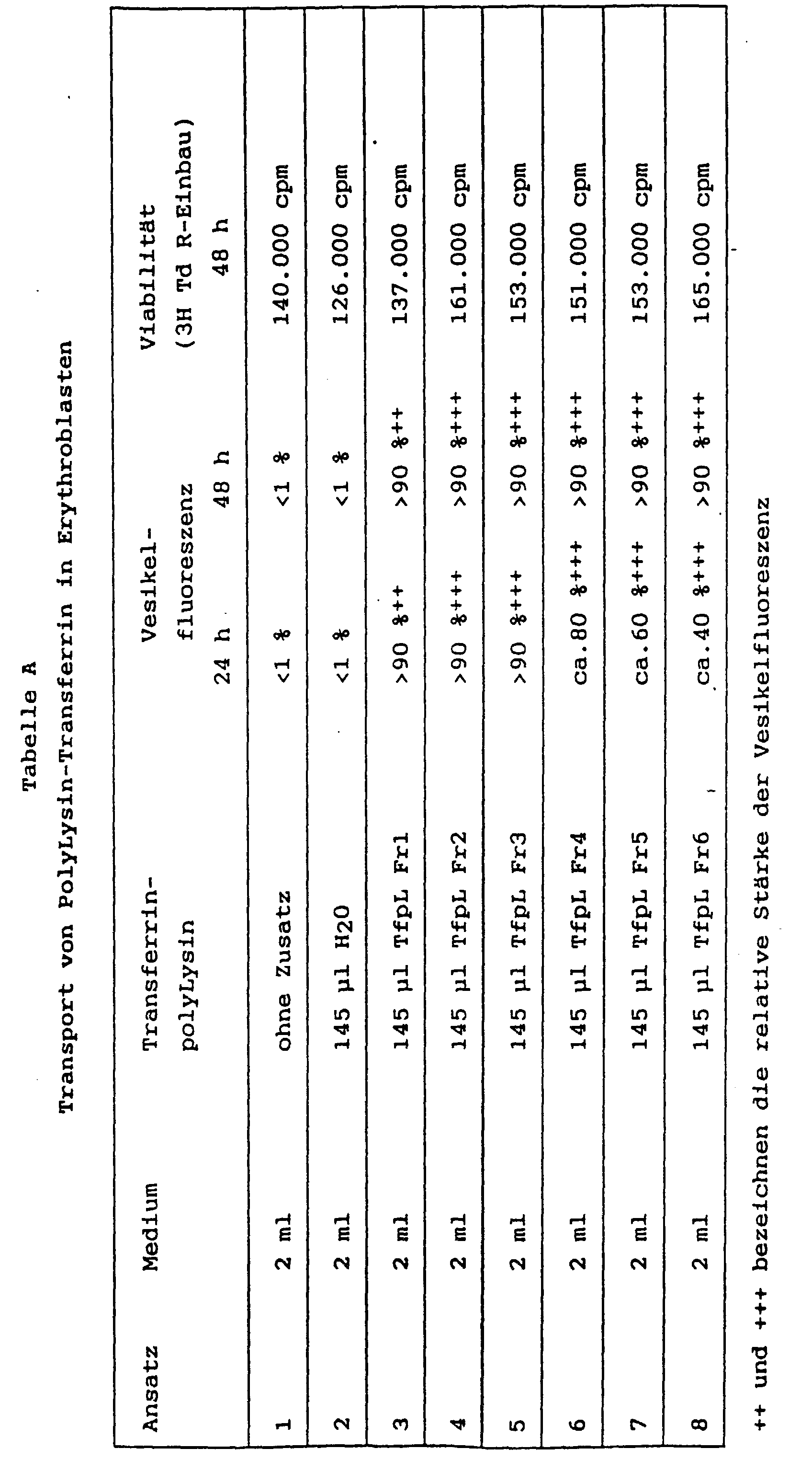

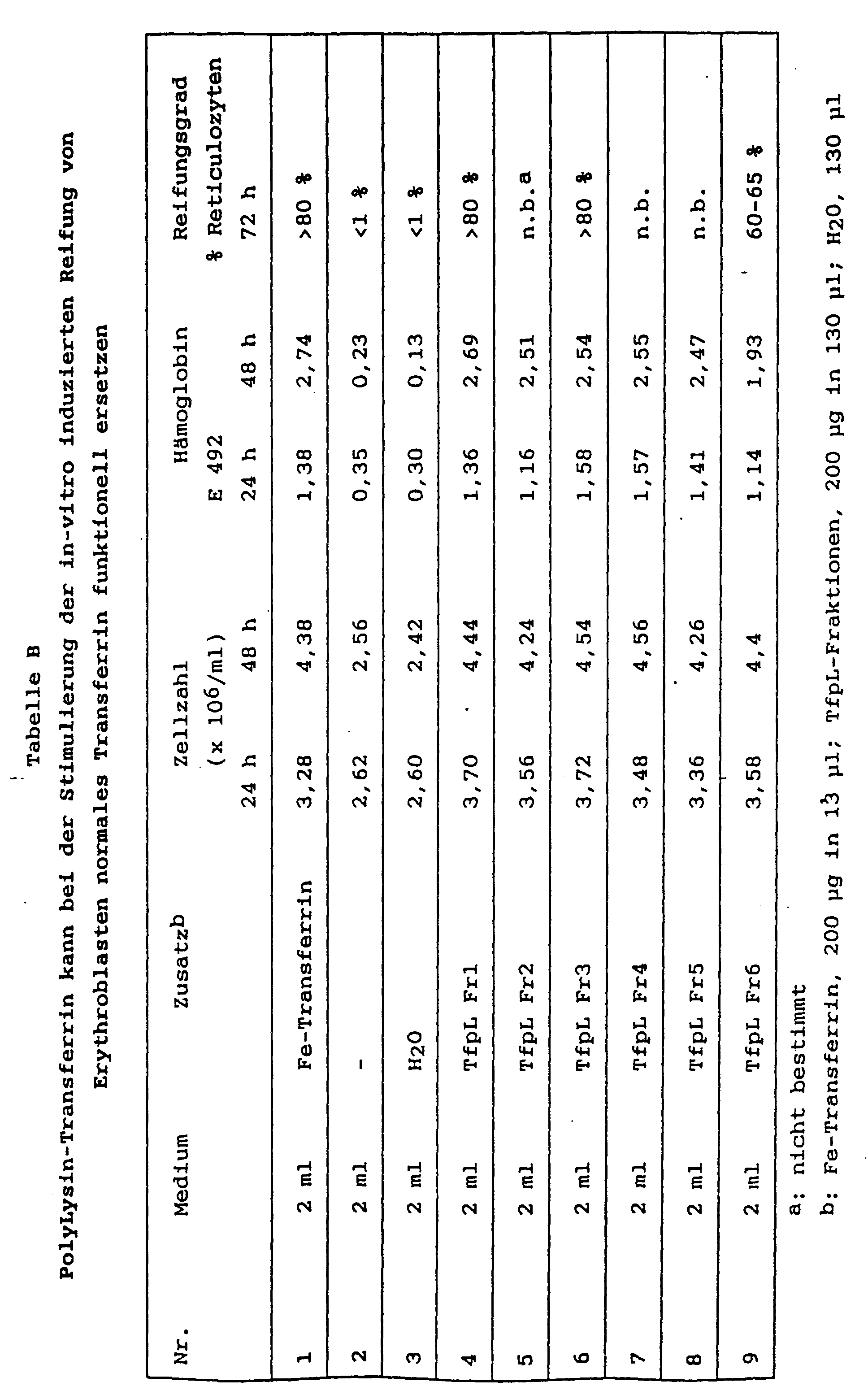

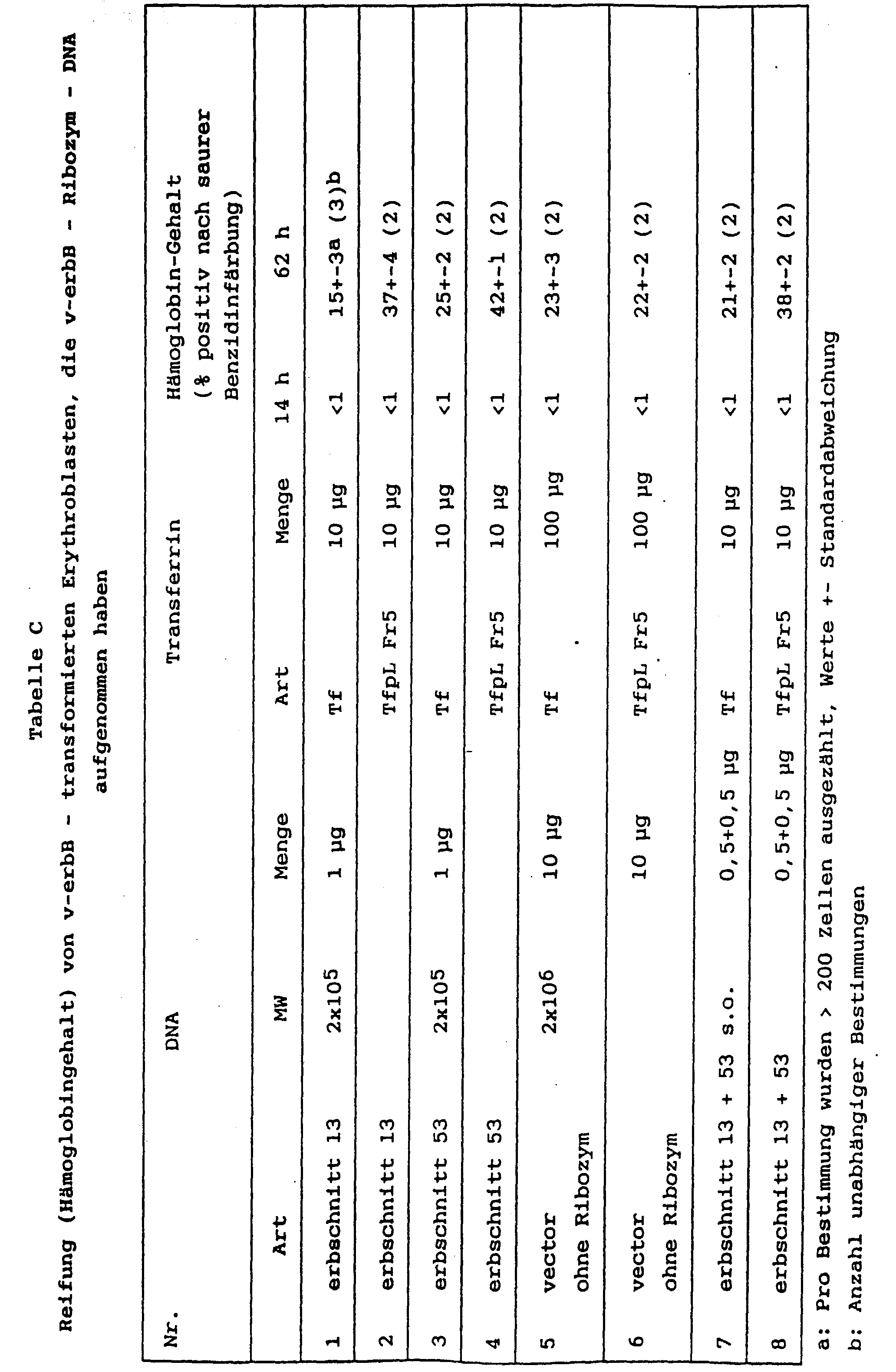

## Patentansprüche

1. DNA-Molekül, gegebenenfalls in Mehrfachkopie vorliegend, enthaltend Abschnitte eines von Polymerase III transkribierten Gens und eine für ein inhibierendes RNA-Molekül kodierende DNA-Sequenz, dadurch gekennzeichnet, daß es die für die Transkription durch Polymerase III erforderlichen Transkriptionseinheiten eines tRNA-Gens einschließlich der für die Sekundärstruktur der tRNA maßgeblichen Sequenzen enthält und daß die für ein inhibierendes RNA-Molekül kodierende DNA-Sequenz innerhalb des DNA-Moleküls derart angeordnet ist, daß das inhibierende RNA-Molekül Teil des Transkripts ist.

2. DNA-Molekül nach Anspruch 1, dadurch gekennzeichnet, daß das inhibierende RNA-Molekül ein Ribozym ist.

3. DNA-Molekül nach Anspruch 2, dadurch gekennzeichnet, daß das Ribozym vom "hammerhead"-Typ ist.

4. DNA-Molekül nach Anspruch 1, dadurch gekennzeichnet, daß das inhibierende RNA-Molekül eine Antisense-RNA ist.

5. DNA-Molekül nach Anspruch 1, dadurch gekennzeichnet, daß es die Transkriptionseinheiten einer Initiations-Met-tDNA enthält.

6. DNA-Molekül nach Anspruch 1, dadurch gekennzeichnet, daß es die Transkriptionseinheiten einer tyr-tDNA enthält.

7. DNA-Molekül nach einem der Ansprüche 1 bis 6, dadurch gekennzeichnet, daß sie die für das inhibierende RNA-Molekül kodierende DNA-Sequenz als Insert zwischen dem A-Block und dem B-Block des tRNA-Gens enthält.

8. DNA-Molekül nach Anspruch 5 oder 7, dadurch gekennzeichnet, daß es die für das inhibierende RNA-Molekül kodierende DNA-Sequenz als Insert in der natürlichen Apal-Restriktionschnittstelle zwischen A- und B-Block enthält.

9. DNA-Molekül nach Anspruch 6, dadurch gekennzeichnet, daß es die für das inhibierende RNA-Molekül kodierende DNA-Sequenz als Insert des Introns enthält.

10. DNA-Molekül nach einem der vorhergehenden Ansprüche, dadurch gekennzeichnet, daß es eine Oligonukleotidsequenz derart enthält, daß die Anticodon-Stammregion des tRNA-Transkripts gegenüber der des Wildtyp-tRNA-Transkripts verlängert ist.

11. DNA-Molekül nach Anspruch 9 oder 10, dadurch gekennzeichnet, daß es die für das inhibierende RNA-Molekül kodierende DNA-Sequenz als Insert in einer künstlich eingeführten Restriktionsschnittstelle enthält.

12. DNA-Molekül nach einem der Ansprüche 9 bis 11, dadurch gekennzeichnet, daß das Intron derart modifiziert ist, daß die Sekundärstruktur der Ausgangs-tRNA stabilisiert wird, wobei die für das Splicing maßgeblichen Strukturen erhalten bleiben.

13. DNA-Molekül nach einem der Ansprüche 1 bis 12, dadurch gekennzeichnet, daß das inhibierende RNA-Molekül gegen virale RNA gerichtet ist.

14. DNA-Molekül nach einem der Ansprüche 1 bis 12, dadurch gekennzeichnet, daß das inhibierende RNA-Molekül gegen Oncogene oder andere Schlüsselgene, die Wachstum und/oder Differenzierung von Zellen kontrollieren, gerichtet ist.

15. DNA-Molekül nach einem der Ansprüche 1 bis 14, dadurch gekennzeichnet, daß es als Mehrfachkopie vorliegt, wobei gleiche oder verschiedene, für ein inhibierendes RNA-Molekül kodierende DNA-Sequenzen enthaltende Untereinheiten als komplette Transkriptionseinheiten vorliegen.

16. Verfahren zum Einführen von DNA-Molekülen gemäß einem der Ansprüche 1 bis 15 in höhere eukaryotische Zellen, dadurch gekennzeichnet, daß die DNA-Moleküle derart modifiziert werden, daß ihre Aufnahme in die Zelle durch Rezeptor-vermittelte Endozytose erfolgt und daß man die Zellen mit den DNA-Molekülen in Berührung bringt.

17. Verfahren nach Anspruch 16, dadurch gekennzeichnet, daß man die DNA-Moleküle mit einem Transferrin-Polykation-Konjugat komplexiert und die Zellen mit diesem Komplex in Berührung bringt.

18. Verwendung der in einem der Ansprüche 1 bis 15 definierten DNA-Moleküle zur Inhibierung von RNA in vivo.

19. Verwendung nach Anspruch 18 zur Inhibierung von Oncogenen oder anderen Schlüsselgenen, die Wachstum und/oder Differenzierung von Zellen kontrollieren.

20. Verwendung nach Anspruch 18 zur Inhibierung von pathogenen Viren.

21. Verwendung nach Anspruch 18 zur Herstellung transgener Tiere, in denen die DNA-Molekül konstitutiv transkribiert wird.

22. Verwendung nach Anspruch 18 zur Herstellung transgener Pflanzen, in denen die DNA-Molekül konstitutiv exprimiert wird.

23. Pharmazeutische Zubereitung, enthaltend als wirksame Komponente ein oder mehrere DNA-Moleküle nach einem der Ansprüche 1 bis 15.

24. Pharmazeutische Zubereitung nach Anspruch 23 zur Inhibierung viraler RNA.

25. Pharmazeutische Zubereitung nach Anspruch 23 zur Inhibierung von Oncogenen oder anderen Schlüsselgenen, die die Wachstumsrate von Zellen kontrollieren.

26. Pharmazeutische Zubereitung nach Anspruch 23 zur Inhibierung der Synthese unerwünschter Genprodukte.

27. Pharmazeutische Zubereitung nach einem der Ansprüche 23 bis 26, dadurch gekennzeichnet, daß sie zusätzlich ein Transferrin-Polykation-Konjugat, das zur Komplexierung des DNA-Moleküls befähigt ist, enthält.

28. Pharmazeutische Zubereitung nach Anspruch 27, dadurch gekennzeichnet, daß sie ein Transferrin-Polylysin-Konjugat enthält.

29. Pharmazeutische Zubereitung nach Anspruch 27, dadurch gekennzeichnet, daß sie ein Transferrin-Protamin-Konjugat enthält.

## Claims

1. DNA molecule, optionally occurring in multiple copies, containing sections of a gene transcribed by polymerase III and a DNA sequence coding for an inhibiting RNA molecule, characterized in that it contains the transcription units of a tRNA gene necessary for transcription by polymerase III, including the sequences which determine the secondary structure of the tRNA, and that the DNA sequence coding for an inhibiting RNA molecule is arranged inside the DNA molecule in such a way that the inhibiting RNA molecule is a part of the transcript.

2. DNA molecule according to claim 1, characterized in that the inhibiting RNA molecule is a ribozyme.

3. DNA molecule according to claim 2, characterized in that the ribozyme is a ribozyme of the hammerhead type.

4. DNA molecule according to claim 1, characterized in that the inhibiting RNA molecule is an antisense RNA.

5. DNA molecule according to claim 1, characterized in that it contains the transcription units of an initiation-met tDNA.

6. DNA molecule according to claim 1, characterized in that it contains the transcription units of a tyr tDNA.

7. DNA molecule according to one of claims 1 to 6, characterized in that it contains the DNA sequence coding for the inhibiting RNA molecule as an insert between the A block and B block of the tRNA gene.

8. DNA molecule according to claims 5 or 7, characterized in that it contains the DNA sequence coding for the inhibiting RNA molecule as an insert in the natural Apa1-restriction cutting site between the A and B blocks.

9. DNA molecule according to claim 6, characterized in that it contains the DNA sequence coding for the inhibiting RNA molecule as an insert in the intron.

10. DNA molecule according to one of the preceding claims, characterized in that it contains an oligonucleotide sequence such that the anticodon stem region of the tRNA transcript is longer than that of the wild-type tRNA transcript.

11. DNA molecule according to claim 9 or 10, characterized in that it contains the DNA sequence coding for the inhibiting RNA molecule as an insert in an artificially introduced restriction cutting site.

12. DNA molecule according to claims 9 to 11, characterized in that the intron is modified so that the secondary structure of the precursor tRNA is stabilized, whilst the structures which are critical for splicing are maintained.

13. DNA molecule according to one of claims 1 to 12, characterized in that the inhibiting RNA molecule is directed against viral RNA.

14. DNA molecule according to one of claims 1 to 12, characterized in that the inhibiting RNA molecule is directed against oncogenes or other key genes which control the growth and/or differentiation of cells.

15. DNA molecule according to one of claims 1 to 14, characterized in that it occurs as a multiple copy, whilst identical or different sub-units containing DNA coding for an inhibiting RNA molecule are present as complete transcription units.

16. Process for introducing DNA molecules according to one of claims 1 to 15 into higher eukaryotic cells, characterized in that the DNA molecules are modified so that they are absorbed into the cell by receptor-mediated endocytosis and the cells are brought into contact with the DNA molecules.

17. Process according to claim 16, characterized in that the DNA molecules are complexed with a transferrinpolycation conjugate and the cells are brought into contact with this complex.

18. Use of the DNA molecules defined in one of claims 1 to 15 for inhibiting RNA in vivo.

19. Use according to claim 18 for inhibiting oncogenes or other key genes which control growth and/or differentiation of cells.

20. Use according to claim 18 for inhibiting pathogenic viruses.

21. Use according to claim 18 for producing transgenic animals in which the DNA molecule is constitutively transcribed.

22. Use according to claim 18 for the preparation of transgenic plants in which the DNA molecule is constitutively expressed.

23. Pharmaceutical preparation containing as active component one or more DNA molecules according to one of claims 1 to 15.

24. Pharmaceutical preparation according to claim 23 for inhibiting viral RNA.

25. Pharmaceutical preparation according to claim 23 for inhibiting oncogenes or other key genes which control the growth rate of cells.

26. Pharmaceutical preparation according to claim 23 for inhibiting the synthesis of undesirable gene products.

27. Pharmaceutical preparation according to one of claims 23 to 26, characterized in that it additionally contains a transferrin-polycation conjugate capable of complexing the DNA molecule.

28. Pharmaceutical preparation according to claim 27, characterized in that it contains a transferrinpolylysine conjugate.

29. Pharmaceutical preparation according to claim 27, characterized in that it contains a transferrinprotamine conjugate.

## Revendications

1. Molécule d'ADN, présente éventuellement sous forme de copies multiples, contenant des segments d'un gène transcrit par la polymérase III et une séquence d'ADN codant une molécule d'ARN inhibitrice, caractérisée en ce qu'elle contient les unités de transcription d'un gène d'ARNt qui sont nécessaires pour la transcription par la polymérase III y compris les séquences déterminantes pour la structure secondaire de l'ARNt et en ce que la séquence d'ADN codant une molécule d'ARN inhibitrice est agencée à l'intérieur de la molécule d'ADN de telle manière que la molécule d'ARN inhibitrice fait partie du transcrit.

2. Molécule d'ADN selon la revendication 1, caractérisée en ce que la molécule d'ARN inhibitrice est un ribozyme.

3. Molécule d'ADN selon la revendication 2, caractérisée en ce que le ribozyme est de type "hammerhead".

4. Molécule d'ADN selon la revendication 1, caractérisée en ce que la molécule d'ARN inhibitrice est un ARN antisens.

5. Molécule d'ADN selon la revendication 1, caractérisée en ce qu'elle contient les unités de transcription d'un Met-ADNt d'initiation.

6. Molécule d'ADN selon la revendication 1, caractérisée en ce qu'elle contient les unités de transcription d'un tyr-ADNt.

7. Molécule d'ADN selon l'une des revendications 1 à 6, caractérisée en ce qu'elle contient la séquence d'ADN codant la molécule d'ARN inhibitrice sous forme d'insert entre le bloc A et le bloc B du gène d'ARNt.

8. Molécule d'ADN selon la revendication 5 ou 7, caractérisée en ce qu'elle contient la séquence d'ADN codant la molécule d'ARN inhibitrice sous forme d'insert dans le site naturel de coupure pour l'enzyme de restriction Apal entre les blocs A et B.

9. Molécule d'ADN selon la revendication 6, caractérisée en ce qu'elle contient la séquence d'ADN codant la molécule d'ARN inhibitrice sous forme d'insert de l'intron.

10. Molécule d'ADN selon l'une des revendications précédentes, caractérisée en ce qu'elle contient une séquence oligonucléotidique de telle manière que la région génératrice d'anticodon du transcrit d'ARNt est prolongée par rapport à celle du transcrit d'ARNt de type sauvage.

11. Molécule d'ADN selon la revendication 9 ou 10, caractérisée en ce qu'elle contient la séquence d'ADN codant la molécule d'ARN inhibitrice sous forme d'insert dans un site de coupure pour une enzyme de restriction introduit artificiellement.

12. Molécule d'ADN selon l'une des revendications 9 à 11, caractérisée en ce que l'intron est modifié de telle manière que la structure secondaire de l'ARNt initial est stabilisée, les structures déterminantes pour l'épissage étant conservées.

13. Molécule d'ADN selon l'une des revendications 1 à 12, caractérisée en ce que la molécule d'ARN inhibitrice est dirigée contre un ARN viral.

14. Molécule d'ADN selon l'une des revendications 1 à 12, caractérisée en ce que la molécule d'ARN inhibitrice est dirigée contre des oncogènes ou d'autres gènes clés qui contrôlent la croissance et/ou la différenciation des cellules.

15. Molécule d'ADN selon l'une des revendications 1 à 14, caractérisée en ce qu'elle est présente sous forme de copies multiples, des sous-unités identiques ou différentes contenant des séquences d'ADN codant une molécule d'ARN inhibitrice étant présentes sous forme d'unités de transcription complètes.

16. Procédé pour introduire des molécules d'ADN selon l'une des revendications 1 à 15 dans des cellules eucaryotiques supérieures, caractérisé en ce que les molécules d'ADN sont modifiées de telle manière que leur introduction dans la cellule est réalisée par endocytose rendue possible par des récepteurs et en ce que l'on met les cellules en contact avec les molécules d'ADN.

17. Procédé selon la revendication 16, caractérisé en ce que l'on complexe les molécules d'ADN avec un conjugué transferrine-polycation et on met les cellules en contact avec ce complexe.

18. Utilisation des molécules d'ADN définies dans l'une des revendications 1 à 15 pour l'inhibition d'ARN in vivo.

19. Utilisation selon la revendication 18 pour l'inhibition d'oncogènes ou d'autres gènes clés qui contrôlent la croissance et/ou la différenciation des cellules.

20. Utilisation selon la revendication 18 pour l'inhibition de virus pathogènes.

21. Utilisation selon la revendication 18 pour l'obtention d'animaux transgéniques dans lesquels la molécule d'ADN est transcrite de manière constitutive.

22. Utilisation selon la revendication 18 pour l'obtention de plantes transgéniques dans lesquelles la molécule d'ADN est exprimée de manière constitutive.

23. Préparation pharmaceutique contenant comme constituant actif une ou plusieurs molécules d'ADN selon l'une des revendications 1 à 15.

24. Préparation pharmaceutique selon la revendication 23 pour l'inhibition d'ARN viral.

25. Préparation pharmaceutique selon la revendication 23 pour l'inhibition d'oncogènes ou d'autres gènes clés qui contrôlent le taux de croissance des cellules.

26. Préparation pharmaceutique selon la revendication 23 pour l'inhibition de la synthèse de produits géniques indésirables.

27. Préparation pharmaceutique selon l'une des revendications 23 à 26, caractérisée en ce qu'elle contient en outre un conjugué transferrine-polycation qui est capable de complexer la molécule d'ADN.

28. Préparation pharmaceutique selon la revendication 27, caractérisée en ce qu'elle contient un conjugué transferrine-polylysine.

29. Préparation pharmaceutique selon la revendication 27, caractérisée en ce qu'elle contient un conjugué transferrine-protamine.
